# EUROPEAN PATENT APPLICATION

(11) **EP 3 586 853 A1**
(43) Date of publication of application: **01.01.2020**
(21) Application number: 17849104.9
(22) Date of filing: 07.09.2017
(51) Int. Cl.: A61K 35/28, C12N 5/0775

(54) **PHARMACEUTICAL COMPOSITION COMPRISING INFLAMMATION-STIMULATED MESENCHYMAL STEM CELL FOR PREVENTION OR TREATMENT OF IMMUNE DISEASE OR INFLAMMATORY DISEASE**

(30) Priority: 07.09.2016 KR 20160115081
(71) Applicant: SCM Lifescience Co., Ltd., Jung-gu Incheon 22332 (KR)
(72) Inventor: SONG, Sun Uk, Incheon 22003 (KR); YI, Tac Ghee, Yangju-si Gyeonggi-do 11481 (KR)
(74) Representative: Vossius & Partner Patentanwälte Rechtsanwälte mbB
(86) International application number: PCT/KR2017/009824
(87) International publication number: WO 2018/048220

(57) **Abstract**

The present disclosure relates to a pharmaceutical composition for preventing or treating immune diseases or inflammatory diseases including inflammatory stimulated mesenchymal stem cells and a method of preparing mesenchymal stem cells for preventing or treating immune diseases or inflammatory diseases. The inflammatory stimulated mesenchymal stem cells of the present disclosure have an effect of releasing acetylcholine, thereby replacing the conventional immunosuppressants and inflammation inhibitors known to have side effects and being useful for the prevention or treatment of immune diseases and inflammatory diseases as a cell therapy agent which can be economically used for the diseases.

## Description

### CROSS-REFERENCE TO RELATED APPLICATIONS

This application is based on and claims priority from Korean Patent Application No. 10-2016-0115081, filed on 09.07.2016, with the Korean Intellectual Property Office, the disclosure of which is incorporated herein in its entirety by reference.

### TECHNICAL FIELD

The present disclosure relates to a pharmaceutical composition for preventing or treating immune diseases or inflammatory diseases including inflammatory stimulated mesenchymal stem cells and a method of preparing mesenchymal stem cells for preventing or treating immune diseases or inflammatory diseases.

### BACKGROUND

Bone marrow, a soft material found inside bones, is the active tissue where new blood cells are produced. It contains at least two types of stem cells. Hematopoietic stem cells (HSCs) are self-renewing stem cells that are produced in all blood passages. Different from HSCs, rare stem cells of nonhematopoietic origin supply and constitute the supportive stromal structure for hematopoiesis. They are capable of differentiating into somatic cells of the mesenchymal lineage. The non-hematopoietic stem cells supporting hematopoiesis are defined as marrow stromal cells or mesenchymal stem cells (MSCs). MSCs have been isolated from a variety of adult tissues and are known to differentiate into not only mesenchymal stem cell types but also non-mesenchymal stem cell types such as neurons. In terms of the differentiation potential, however, although MSCs possess the inherent multipotency, their uncertain capacity for *in vivo* transdifferentiation is likely to be of limited utility for clinical applications.

So far, many compound immunosuppressants or anti-inflammatory agents have been developed and clinically most commonly used immunosuppressants include cyclosporine (Neoral, Cipol A), azathioprine (imuran), and prednisolone (a kind of steroid). The immunosuppressive agent inhibits several processes such as antigen phagocytosis by macrophage, antigen recognition by lymphocyte, cell division, division of T cells and B cells, and antibody production during the course from antigen stimulation to antibody production, causing immunosuppression. Most of them have antitumor activity because they inhibit cell division by mediating DNA disorder, DNA synthesis inhibition and the like. However, there have been problems such as hypertension and nephrotoxicity (lowered renal function), which are typical side effects, and that the incidence of this side effect is high, so it is necessary to observe the progress sufficiently on use. Other side effects include tremors, crisis, hepatitis, bile retention, increased blood uric acid, decreased muscle strength, hypertrichosis, gingival hypertrophy and the like. Azathioprine, a commonly used inhibitor, may develop complications such as an inhibitory function of bone marrow such as decreased leukocyte levels, anemia, and thrombocytopenia as well as pancreatitis, hepatitis, and bile retention and rare complications such as hair loss and fever. Prednisolone, which is one of the steroids, started to be used first among immunosuppressants, but it is a drug to be cautious because it not only accelerates arteriosclerosis but also causes hypertension, gastric ulcer, diabetes, growth inhibition, osteoporosis, cataract, and glaucoma. Thus, there is a need for a safe inhibitor or an anti-inflammatory agent.

### SUMMARY

Bone marrow, a soft material found inside bones, is the active tissue where new blood cells are produced. It contains at least two types of stem cells. Hematopoietic stem cells (HSCs) are self-renewing stem cells that are produced in all blood passages. Different from HSCs, rare stem cells of nonhematopoietic origin supply and constitute the supportive stromal structure for hematopoiesis. They are capable of differentiating into somatic cells of the mesenchymal lineage. The non-hematopoietic stem cells supporting hematopoiesis are defined as marrow stromal cells or mesenchymal stem cells (MSCs). MSCs have been isolated from a variety of adult tissues and are known to differentiate into not only mesenchymal cell types but also non-mesenchymal cell types such as neurons. In terms of the differentiation potential, however, although MSCs possess the inherent multipotency, their uncertain capacity for *in vivo* transdifferentiation is likely to be of limited utility for clinical applications.

So far, many compound immunosuppressants or anti-inflammatory agents have been developed and clinically most commonly used immunosuppressants include cyclosporine (Neoral, Cipol A), azathioprine (imuran), and prednisolone (a kind of steroid). The immunosuppressant inhibits several processes such as antigen phagocytosis by macrophage, antigen recognition by lymphocyte, cell division, division of T cells and B cells, and antibody production during the course from antigen stimulation to antibody production, thereby causing immunosuppression. Most of them have antitumor activity because they inhibit cell division by mediating DNA disorder, DNA synthesis inhibition and the like. However, there have been problems such as hypertension and nephrotoxicity (lowered renal function), which are typical side effects, and that the incidence of this side effect is high, so it is necessary to observe the progress sufficiently on use. Other side effects rarely include tremors, crisis, hepatitis, bile retention, increased blood uric acid, decreased muscle strength, hypertrichosis, gingival hypertrophy and the like. Azathioprine, a commonly used inhibitor, may develop complications such as an inhibitory function of bone marrow such as decreased leukocyte levels, anemia, and thrombocytopenia as well as pancreatitis, hepatitis, and bile retention and rare complications such as hair loss and fever. Prednisolone, which is one of the steroids, started to be used first among immunosuppressants, but it is a drug to be cautious because it not only accelerates arteriosclerosis but also causes hypertension, gastric ulcer, diabetes, growth inhibition, osteoporosis, cataract, glaucoma, and the like. Thus, there is a need for a safe immunosuppressant or an anti-inflammatory agent.

In order to solve the above problems, an exemplary embodiment of the present disclosure provides a pharmaceutical composition for preventing or treating immune diseases or inflammatory diseases including inflammatory stimulated mesenchymal stem cells.

Another exemplary embodiment of the present disclosure provides a method of preparing mesenchymal stem cells for preventing or treating immune diseases or inflammatory diseases, the method including culturing mesenchymal stem cells with inflammatory stimulation.

According to the exemplary embodiments of the present disclosure, the inflammatory stimulated mesenchymal stem cells of the present disclosure have an effect of releasing acetylcholine, thereby replacing the conventional immunosuppressants and inflammation inhibitors, and being useful for the prevention or treatment of immune diseases and inflammatory diseases as a cell therapy agent which can be economically used for the diseases.

The foregoing summary is illustrative only and is not intended to be in any way limiting. In addition to the illustrative aspects, embodiments, and features described above, further aspects, embodiments, and features will become apparent by reference to the drawings and the following detailed description.

### BRIEF DESCRIPTION OF THE DRAWINGS

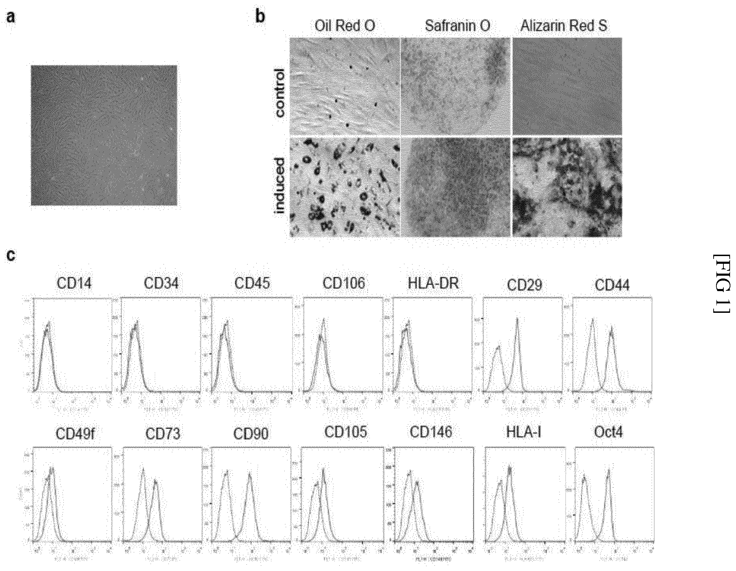

FIG. 1A illustrates the result of confirming that the MSCs of the present disclosure have a fibroblast-like morphology. FIG. 1B illustrates the results of identifying the adipogenically, chondrogenically and osteogenically differentiating potentials of the MSCs of the present disclosure. FIG. 1C is a graph illustrating the results of flow cytometry analysis of the positive or negative marker of the MSCs of the present disclosure.

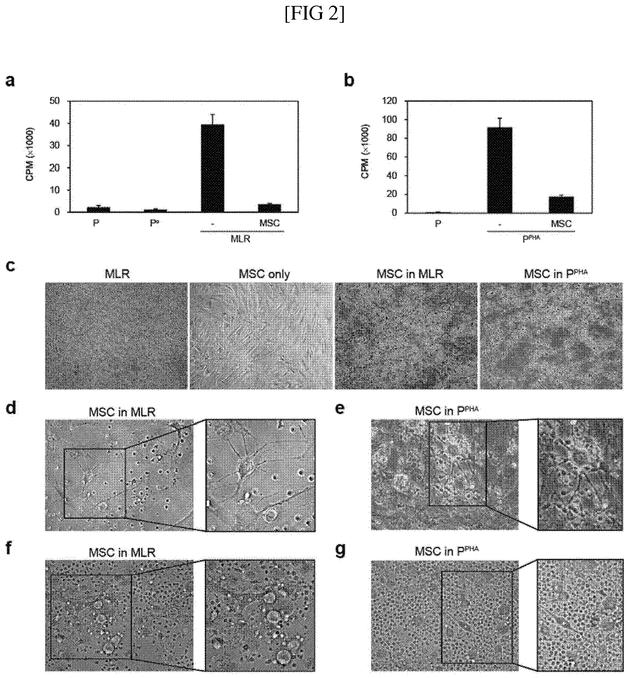

FIG. 2A illustrates the result of confirming an alloantigenic immune response through the lymphocyte proliferation and activation for a mixed lymphocyte reaction (MLR) and PBMCs (P and Po) with or without MSCs. FIG. 2B illustrates the results of lymphocyte proliferation and activation of PBMCs (P) and human PBMCs treated with PHA (1 µg/ml) (P^{PHA}) with or without co-culture of MSCs. FIG. 2C illustrates the result of observing abnormal morphological changes in MSCs when the MSCs were co-cultured with MLR- or PHA-activated PBMCs (P^{PHA}) using an optical microscope. FIGS. 2D and 2E show the results of confirming neuron-like morphological properties after co-culturing MSCs in MLR- or PHA-activated PBMCs(P^{PHA}), respectively. FIGS. 2F and 2G illustrate the results of confirming neurosphere-like cell clusters (red arrows) after co-culturing MSCs in MLR- or PHA-activated PBMCs(P^{PHA}), respectively.

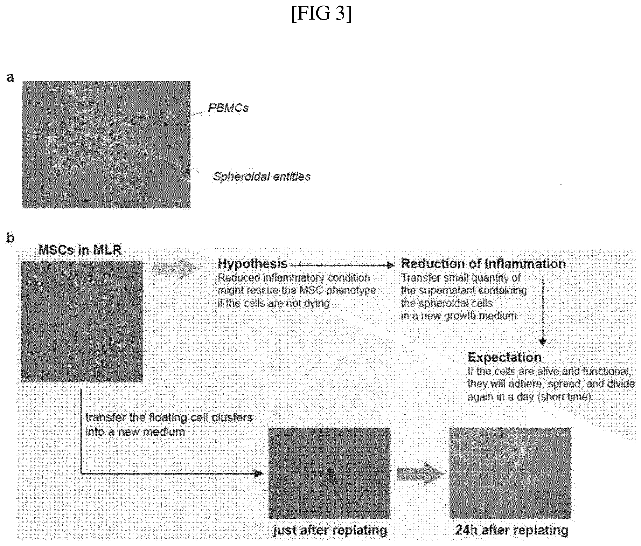

FIG. 3A illustrates the results of confirming the generation of spheroid entities in MSCs for inflammatory conditions. FIG. 3B illustrates the results of confirming the generation of spheroid entities and MSCs properties of MSCs when the inflammatory condition is minimized.

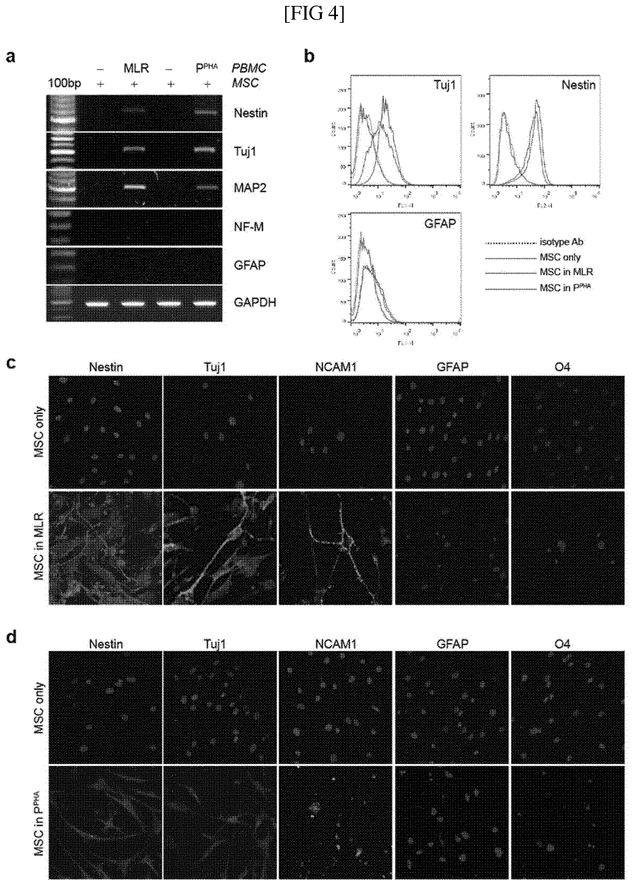

FIG. 4A illustrates the results of confirming the expression of nestin, Tuj1, MAP2, NF-M and GFAP by performing semi-quantitative RT-PCR after co-culturing MSCs in MLR- or PHA-activated PBMCs. FIG. 4B illustrates the results of flow cytometry analysis of nestin, Tuj1 and GFAP after co-culturing MSCs in MLR- or PHA-activated PBMCs. FIGS. 4C and 4D illustrate the results of confirming the expression levels of nestin, Tuj1, NCAM1, GFAP and 04 by immunofluorescence staining after co-culturing MSCs in MLR- or PHA-activated PBMCs, respectively.

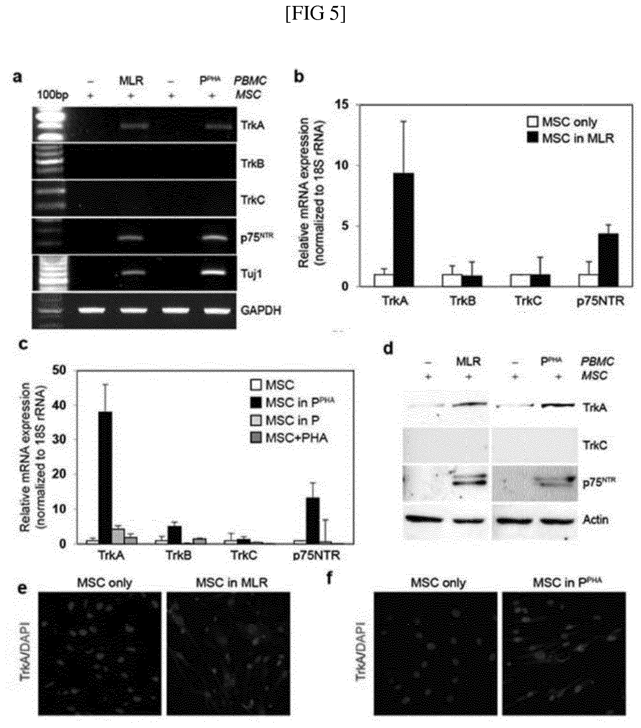

FIG. 5A illustrates the results of confirming the expression of TrkA, TrkB, TrkC and p75^{NTR} by performing semi-quantitative RT-PCR after co-culturing MSCs in MLR- or PHA-activated PBMCs. FIGS. 5B and 5C illustrate the results of confirming the expression of TrkA, TrkB, TrkC and p75^{NTR} by qRT-PCR after co-culturing MSCs in MLR- or PHA-activated PBMCs. FIG. 5D illustrates the results of confirming the expression of TrkA, TrkB, TrkC and p75^{NTR} by performing Western blotting after co-culturing MSCs in MLR- or PHA-activated PBMCs. FIGS. 5E and 5F illustrate the results of confirming the expression levels of TrkA by immunofluorescence staining after co-culturing MSCs in MLR- or PHA-activated PBMCs, respectively.

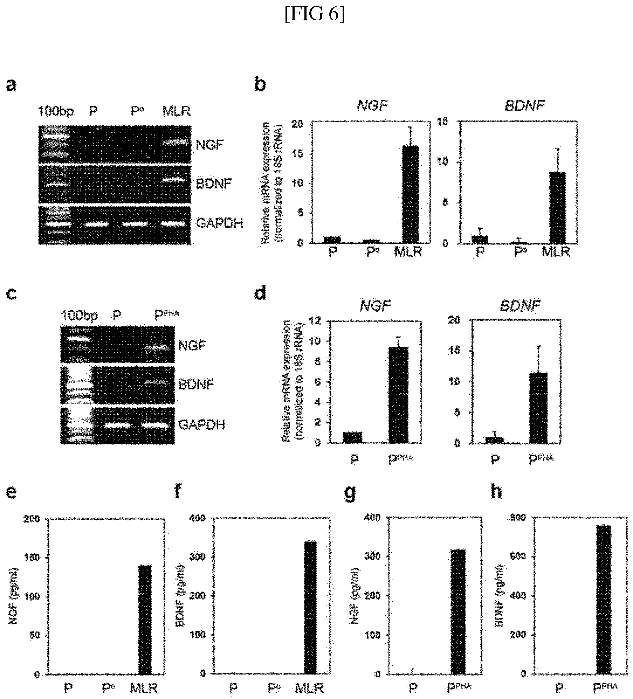

FIG. 6A illustrates the results of confirming the expression levels of NGF and BDNF by performing semi-quantitative RT-PCR on MLR-activated PBMCs. FIG. 6B illustrates the results of confirming the expression levels of NGF and BDNF by performing qRT-PCR on MLR-activated PBMCs. FIG. 6C illustrates the results of confirming the expression levels of NGF and BDNF by performing semi-quantitative RT-PCR on PHA-activated PBMCs. FIG. 6D illustrates the results of confirming the expression levels of NGF and BDNF by performing qRT-PCR on PHA-activated PBMCs. FIGS. 6E and 6H illustrate the results of confirming the degree of release amount of NGF and BDNF released from MLR- or PHA-activated PBMCs, respectively, by performing ELISA.

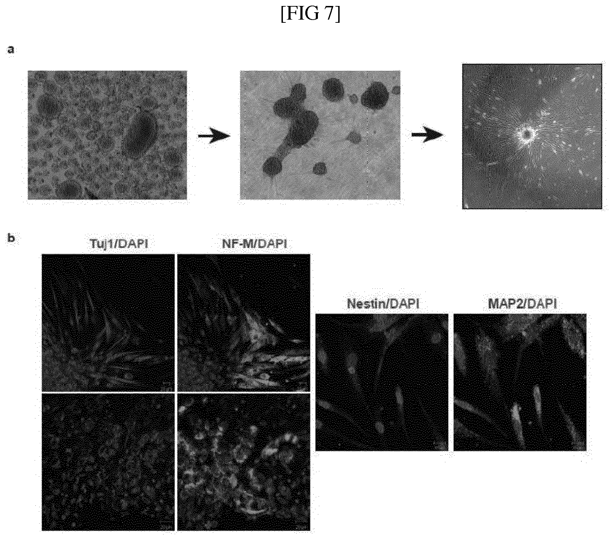

FIG. 7A illustrates the results of confirming the formation of spheroids of MSCs under inflammatory conditions. FIG. 7B illustrates the results of confirming the expression levels of Tuj1, NF-M and MAP2 of the spheroid of MSCs.

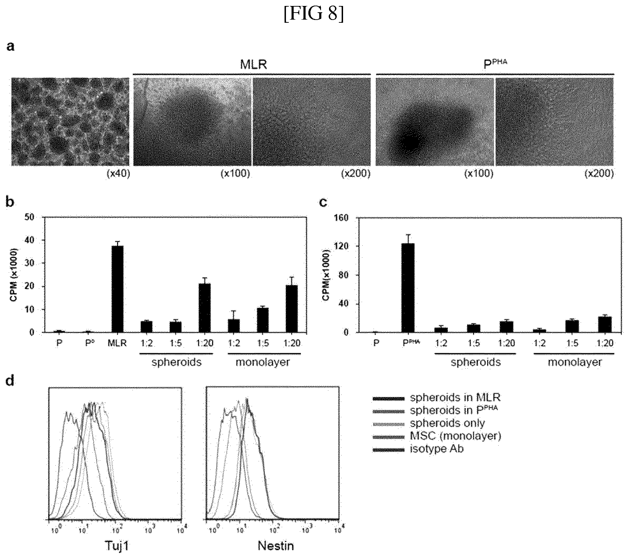

FIG. 8A illustrates the results of confirming the formation of spheroids of PBMCs activated under inflammatory conditions. FIG. 8B illustrates the result of confirming the degree of inhibition of lymphocyte proliferation according to the ratio (1 : 2, 1 : 5 and 1 : 20) of the MSCs spheroid or MSCs monolayer under the condition of mixed lymphocyte reaction (MLR). FIG. 8C illustrates the results of confirming the degree of inhibition of lymphocyte proliferation according to the ratio (1 : 2, 1 : 5 and 1 : 20) of the MSCs spheroid or MSCs monolayer under the condition of phytohemagglutinin (PHA). FIG. 8D illustrates the results of flow cytometry analysis of the expression of Tuj1 and nestin of MSCs spheroid under the inflammatory condition (mixed lymphocyte reaction (MLR) condition or phytohemagglutinin (PHA) treatment condition).

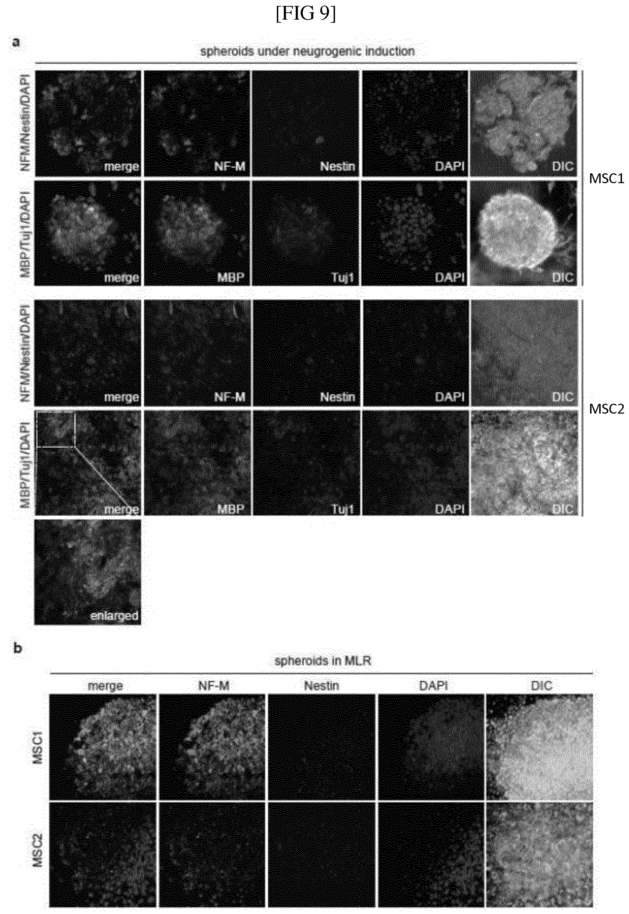

FIG. 9A illustrates the results of confirming the expression of Tuj1, nestin, MBP and NF-M after culturing non-treated MSCs in a neurobasal medium. FIG. 9B illustrates the results of confirming expression of Tuj1, nestin, MBP and NF-M after culturing MSCs under mixed lymphocyte reaction (MLR) conditions in a neurobasal medium.

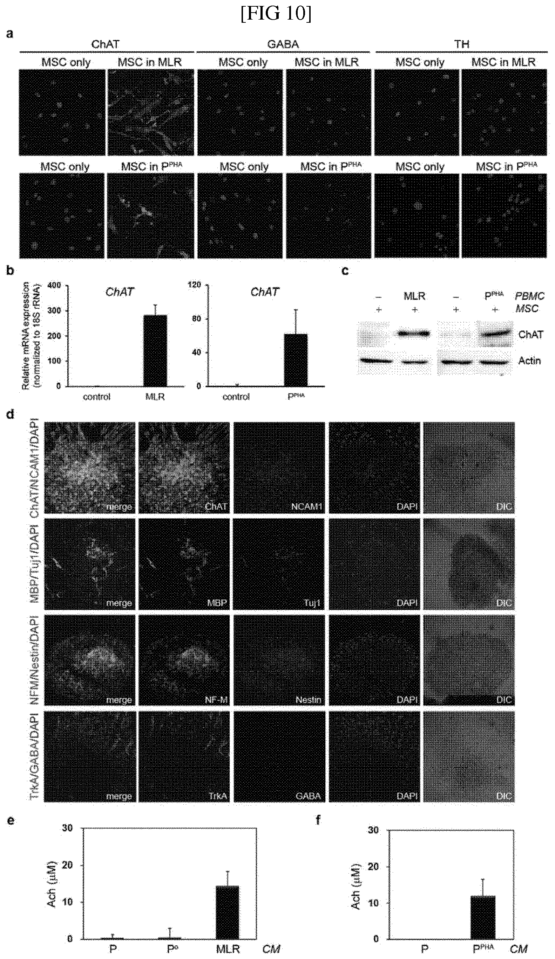

FIG. 10A illustrates the results of confirming the expression levels of ChAT, TH and GABA by immunofluorescence staining of MSCs after co-culturing MSCs in MLR-activated or PHA-activated PBMCs. FIG. 10B illustrates the results of confirming the expression levels of ChAT by performing qRT-PCR after co-culturing MSCs in MLR- or PHA-activated PBMCs. FIG. 10C illustrates the results of confirming the expression levels of ChAT by performing Western blotting after co-culturing MSCs in MLR- or PHA-activated PBMCs. FIG. 10D illustrates the results of confirming the expression levels of ChAT, NCAM1, MBP, Tuj1, NF-M, nestin, TrkA and GABA by immunofluorescence staining after co-culturing MSCs in MLR-activated PBMCs. FIG. 10E illustrates the results of confirming acetylcholine release compared to PBMCs only group (P and Po) after co-culturing MSCs in MLR-activated PBMCs. FIG. 10F illustrates the results of confirming acetylcholine release compared to PBMCs only group (P) after co-culturing MSCs in PHA-activated PBMCs.

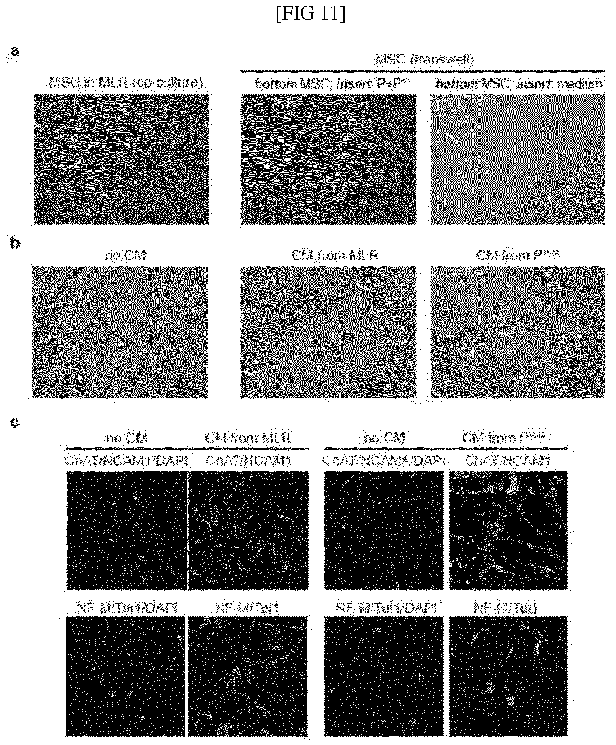

FIG. 11A illustrates the results of confirming the change of MSCs into cholinergic neuron-like phenotype under inflammatory conditions, as compared to the normal culture or the transwell plate culture. FIG. 11B illustrates the result of confirming the change of MSCs into cholinergic neuron-like phenotype under conditioned medium (CM). FIG. 11C illustrates the results of confirming expression of ChAT, NCAM1, NF-M and Tuj1 under the condition as illustrated in FIG. 11B.

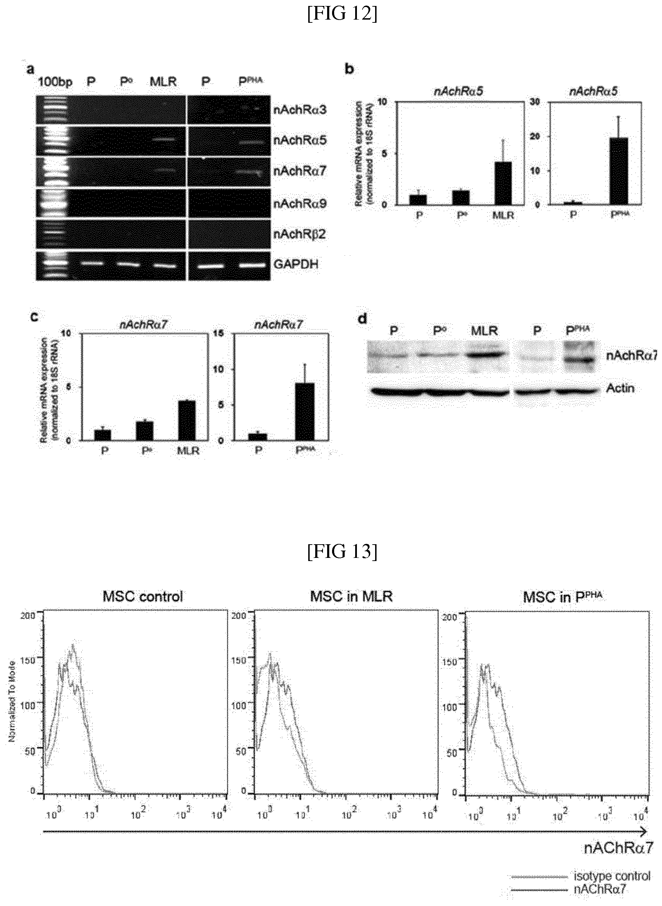

FIG. 12A illustrates the result of confirming the expression levels of nAChRα3, nAChRα5, nAChRα7, nAChRα8 and nAChRβ2 compared to PBMCs only group (P and Po) by performing semi-quantitative RT-PCR after co-culturing MSCs in MLR- or PHA-activated PBMCs. FIG. 12B illustrates the result of confirming the expression levels of nAChRα5 compared to PBMCs only group (P and Po) by performing qRT-PCR after co-culturing MSCs in MLR- or PHA-activated PBMCs. FIG. 12C illustrates the result of confirming the expression levels of nAChRα7 compared to PBMCs only group (P and Po) by performing qRT-PCR after co-culturing MSCs in MLR- or PHA-activated PBMCs. FIG. 12D illustrates the result of confirming the expression levels of nAChRα7 compared to PBMCs only group (P and Po) by performing Western blotting after co-culturing MSCs in MLR- or PHA-activated PBMCs.
FIG. 13 illustrates the results of flow cytometry analysis of PBMCs cultured in inflammatory conditions (mixed lymphocyte reaction (MLR) condition or phytohemagglutinin (PHA) treatment condition).

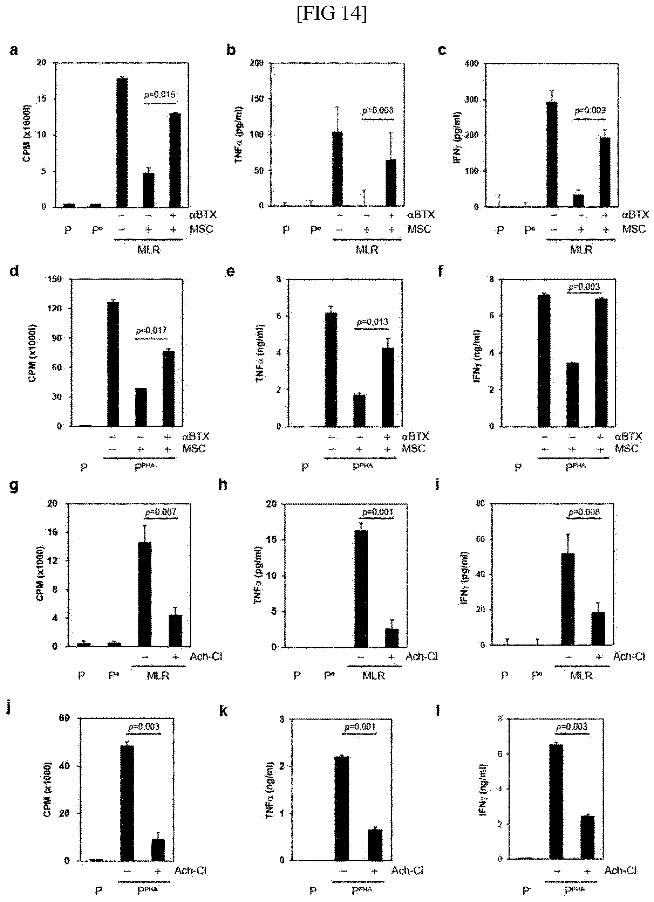

FIGS. 14A to 14C illustrate the results of confirming each lymphocyte proliferation, TNF-α and IFN-γ compared to PBMCs only group (P and Po), after co-culturing MSCs with MLR-activated PBMCs in the medium supplemented with α-BTX. FIGS. 14D to 14F illustrate the results of confirming each lymphocyte proliferation, TNF-α and IFN-γ compared to PBMCs only group (P and Po), after co-culturing MSCs with PHA-activated PBMCs in the medium supplemented with a-BTX. FIGS. 14G to 14I illustrate the results of confirming each lymphocyte proliferation, TNF-α and IFN-γ compared to PBMCs only group (P and Po), after co-culturing MSCs with MLR-activated PBMCs in the medium supplemented with ACh-Cl. FIGS. 14J to 14L illustrate the results of confirming each lymphocyte proliferation, TNF-α and IFN-γ compared to PBMCs only group (P and Po), after co-culturing MSCs with PHA-activated PBMCs in the medium supplemented with ACh-Cl.

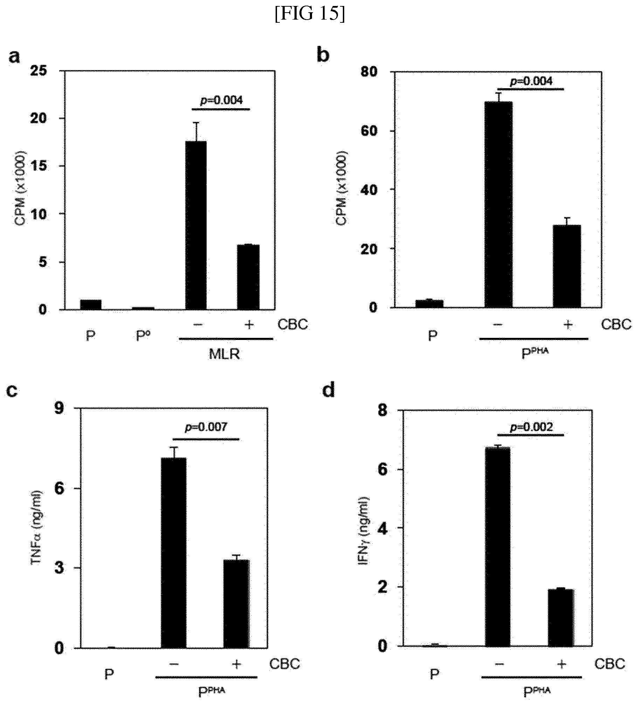

FIGS 15A to 15D illustrate the results of confirming each lymphocyte proliferation, TNF-α and IFN-γ compared to PBMCs only group (P and Po), after co-culturing MSCs with MLR- or PHA-activated PBMCs in the medium supplemented with carbachol which is a non-specific cholinergic agonist.

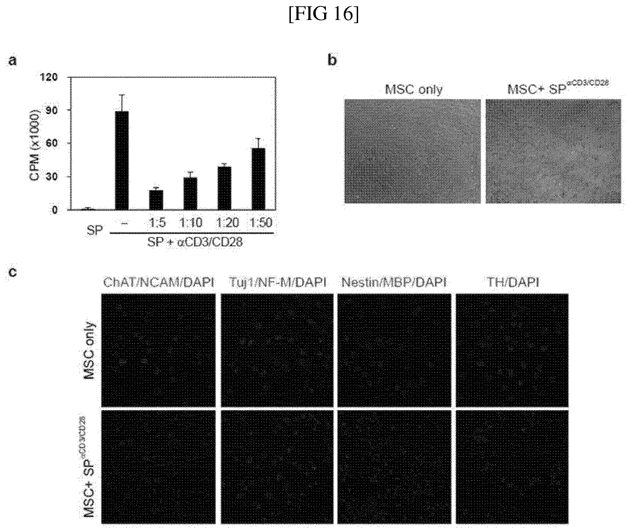

FIG. 16A illustrates the results of confirming inhibition of lymphocyte proliferation after culturing CD3/CD28-activated mouse splenocytes (2 × 10⁵) and MSCs (4 × 10³ to 4 × 10⁴) at a ratio of 1 : 5, 1 : 10, 1 : 20 and 1 : 50. FIG. 16B illustrates the result of confirming whether cholinergic neuron-like phenotypic changes of mouse splenocyte-treated MSCs. FIG. 16C illustrates the results of confirming the expression of ChAT, NCAM1, Tuj1, NF-M, nestin, MBP and TH of mouse splenocyte-treated MSCs.

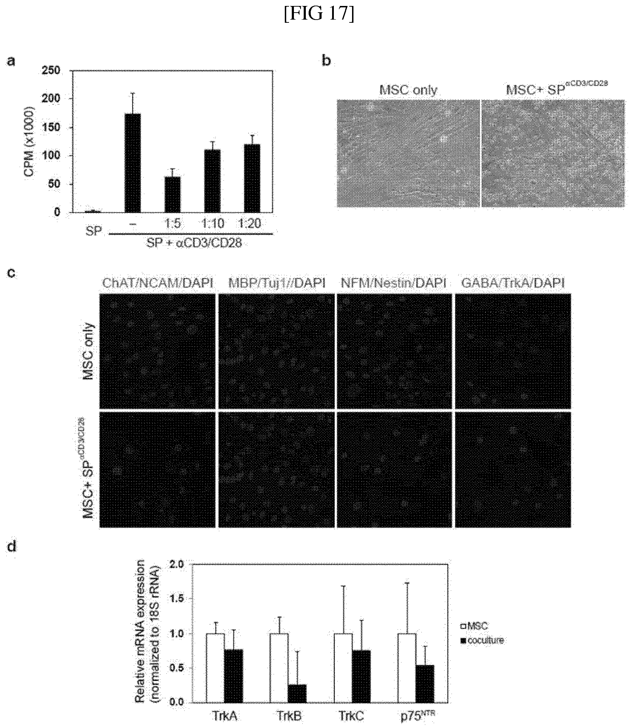

FIG. 17A illustrates the results of confirming inhibition of lymphocyte proliferation after culturing CD3/CD28-activated rat splenocytes (2 × 10⁵) and MSCs (4 × 10³ to 4 × 10⁴) at a ratio of 1 : 5, 1 : 10 and 1 : 20. FIG. 17B illustrates the result of confirming whether cholinergic neuron-like phenotypic changes of rat splenocyte-treated MSCs. FIG. 17C illustrates the results of confirming the expression of ChAT, NCAM1, Tuj1, NF-M, nestin, TH and GABA of mouse splenocyte-treated MSCs. FIG. 17D illustrates the results of comparing expression change in mRNA of TrkA, TrkB, TrkC and p75^{NTR} which are nerve growth factor receptors.

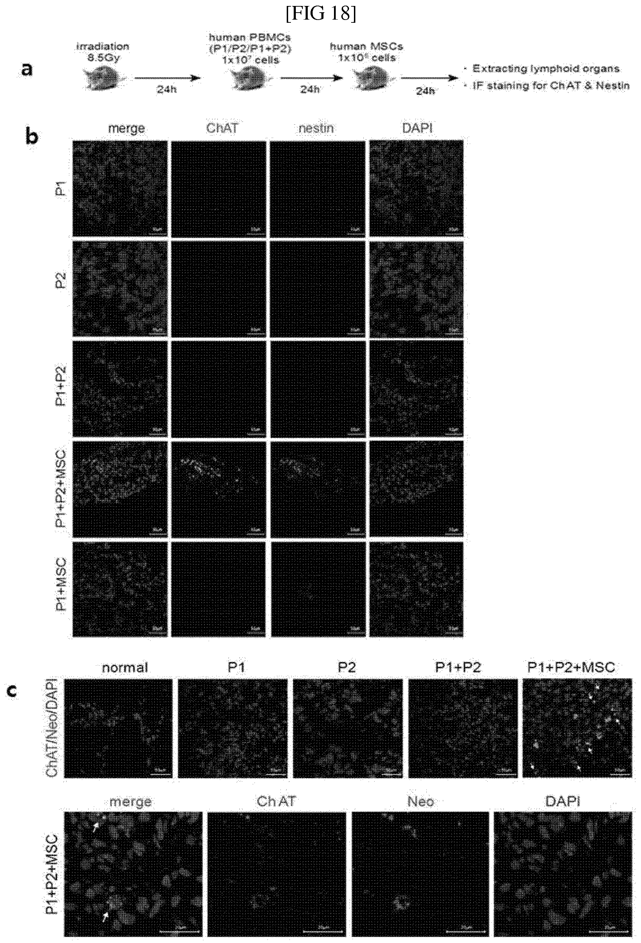

FIG. 18A is a schematic view of the process of confirming ChAT+ nestin+ human cells by inoculating PBMCs and MSCs into a transient humanized GVHD mouse model. FIG. 18B illustrates the expression levels of anti-ChAT and anti-nestin antibodies in each of human MSCs-treated mice which are injected with PBMCs (PI or P2) or mixed PBMCs (P1 + P2) in order to identify ChAT+ nestin+ human cells. FIG. 18C illustrates the results of injecting biocompatible silica-coated fluorescent nanoparticles into human MSCs-treated mice which are injected with PBMCs (PI or P2) or mixed PBMCs (PI + P2) in order to identify whether or not MSC cytoplasm (The arrow indicates the MSC cytoplasm).

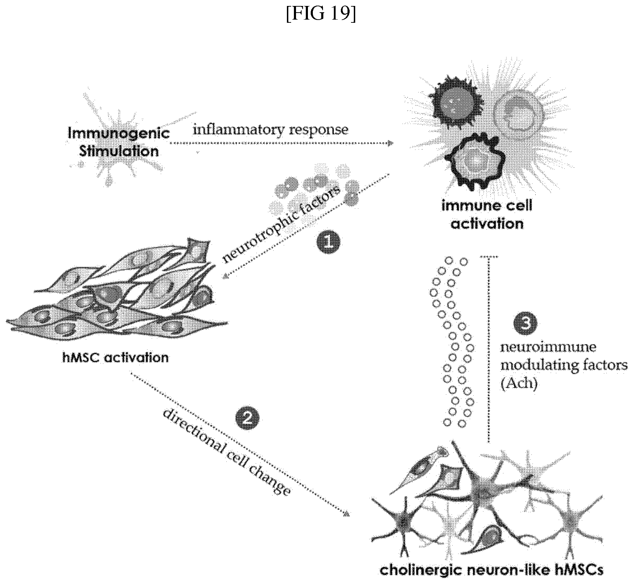

FIG. 19 is a schematic view of illustrating the cholinergic anti-inflammatory mechanism of MSCs for the inflammatory stimulus of the present disclosure.

### DETAILED DESCRIPTION

In the following detailed description, reference is made to the accompanying drawing, which forms a part hereof. The illustrative embodiments described in the detailed description, drawing, and claims are not meant to be limiting. Other embodiments may be utilized, and other changes may be made, without departing from the spirit or scope of the subject matter presented here.

The present disclosure provides a pharmaceutical composition for preventing or treating immune diseases or inflammatory diseases including inflammatory stimulated mesenchymal stem cells.

The mesenchymal stem cells can be isolated by a subfractionation culturing method, and the mesenchymal stem cells of the present disclosure isolated by the above method can express CD29, CD44, CD49f, CD73, CD90, CD105, CD146, HLA-class I (HLA-I) and Oct4.

In the present disclosure, the term "subfractionation culturing method" refers to a method in which bone marrow is collected from a subject and cultured, only the culture supernatant is transferred to a new vessel and cultured, then the culture supernatant produced in the culture is separated, followed by repeated cultures in the culture containers treated with or without coating agents, thereby obtaining mesenchymal stem cells. The contents described in Korean Patent No. 10-0802011 may be incorporated herein in its entirety by reference, and the subfractionation culturing method may be preferably carried out as disclosed in the above document.

The inflammatory stimulated mesenchymal stem cells are ones cultured under at least one culture condition selected from the group consisting of mixed lymphocyte reaction (MLR), mitosis-promoting substance (mitogen) treatment and cytokine treatment conditions. The mitosis-promoting substance includes at least one selected from the group consisting of phytohemagglutinin (PHA), concanavalin A (Con A), pokeweed mitogen (PWM), lipopolysaccharide, streptolysin S, a mercury compound and an anti-lymphocyte antibody, but is not limited thereto.

In the present disclosure, the term "mixed lymphocyte reaction (MLR)" is one of the methods of measuring cellular immune function *in vitro* and used to examine histocompatibility between donor and recipient for organ transplantation. When lymphocytes of the donor and the recipient are mixed and cultured, the lymphocytes begin to divide if the two histocompatibility antigens are different. The division occurrence can be determined according to whether or not 3H-thymidine previously loaded on the medium has been received in the DNA. In the present disclosure, the mixed lymphocyte reaction refers to culturing the respective PBMCs obtained from different donors together. The mesenchymal stem cells are then treated therewith so as to obtain the inflammatory stimulated mesenchymal stem cells of the present disclosure.

In the present disclosure, the term "mitosis-promoting substance (mitogen)" refers to a substance which causes cell division and immunologically means one which is non-specific to antigen (multi-clonal) to ghost lymphocytes, thereby causing the division.

In the present disclosure, the term "phytohemagglutinin (PHA)" refers to a lectin (sugar-binding protein) having an aggregation activity of cells derived from a plant and has a characteristic of acting on T cells only. In the present disclosure, the phytohemagglutinin treatment means treating PBMCs obtained from a donor with PHA and then treating the resultants with the mesenchymal stem cells. The inflammatory stimulated mesenchymal stem cells of the present disclosure may be obtained by this treatment.

In the present disclosure, the term "Concanavalin A (Con A)" is one of the crystalline proteins obtained from the seeds of the pea (*Canavalia ensiformis*). Con A activates T cells but does not activate B cells so that if insolubilized, B cells are also activated. Further, since some cancer cells show high agglutinability to Con A compared with normal cells, Con A has been used as a means of studying the specificity of cancer cell membrane structure.

In the present disclosure, the term "inflammatory stimulated mesenchymal stem cell" refers to a mesenchymal stem cell having properties similar to neurons, which is inflammatory-stimulated by the mixed lymphocyte reaction (MLR), mitosis-promoting substance (mitogen) treatment and cytokine treatment conditions and preferably, the mixed lymphocyte reaction (MLR) or phytohemagglutinin (PHA) condition. In one embodiment of the present disclosure, it was confirmed that the inflammatory stimulated mesenchymal stem cells of the present disclosure are changed into neuron-like morphology. Their morphological changes are accompanied by expression of neuronal markers, including Tuj1, nestin and MAP2, but not glial markers, such as an astrocyte marker GFAP and an oligodendrocyte marker 04.

The inflammatory stimulated mesenchymal stem cells are changed into a cholinergic neuron-like phenotype and can release acetylcholine.

In the present disclosure, the term "cholinergic neuron" refers to a neuron that releases acetylcholine as a chemical delivery substance from the end of a nerve fiber.

In the present disclosure, the term "acetylcholine" is a neurotransmitter that is an ester of choline and acetic acid, is secreted at the end of the nerve, and is a chemical substance that transmits nerve stimulation to muscles.

The present composition may further include an agonist of acetylcholine and may include at least one selected from the group consisting of ACh chloride (ACh-Cl), carbachol, epibatidine, dimethylphenylpiperazinium (DMPP), suxamethonium, cytisine, nicotine, nifene, varenicline, 3,4-methylenedioxymethamphetamine (MDMA), and methamphetamine. It is not limited thereto as long as it is for the prevention or treatment of immune diseases or inflammatory diseases.

The immune diseases or inflammatory diseases may include at least one selected from the group consisting of autoimmune diseases, transplant rejection, arthritis, graft-versus-host-disease, bacterial infection, sepsis and inflammation.

The autoimmune diseases may, but be limited to, include at least one selected from the group consisting of Crohn's disease, erythema, atopic dermatitis, rheumatoid arthritis, Hashimoto's thyroiditis, pernicious anemia, Addison's disease, type 1 diabetes, lupus, chronic fatigue syndrome, fibromyalgia, hypothyroidism, hyperthyroidism, scleroderma, Behcet's disease, inflammatory bowel disease, multiple sclerosis, myasthenia gravis, Meniere's syndrome, Guillain-Barre syndrome, Sjogren's syndrome, vitiligo, endometriosis, psoriasis, vitiligo, systemic scleroderma, asthma and ulcerative colitis.

The pharmaceutical compositions of the present disclosure may further include suitable carriers, excipients and diluents conventionally used in the manufacture of pharmaceutical compositions. Further, solid or liquid pharmaceutical additives may be used for the preparation of pharmaceutical compositions. The preparation additive may be either organic or inorganic.

Examples of the excipient include lactose, sucrose, white sugar, glucose, corn starch, starch, talc, sorbitol, crystalline cellulose, dextrin, kaolin, calcium carbonate, silicon dioxide and the like. Examples of the binder may include polyvinyl alcohol, polyvinyl ether, ethylcellulose, methylcellulose, gum arabic, tragacanth, gelatin, shellac, hydroxypropyl cellulose, hydroxypropylmethyl cellulose, calcium citrate, dextrin, pectin, and the like. Examples of the lubricant include magnesium stearate, talc, polyethylene glycol, silica, and hydrogenated vegetable oil. Any coloring agent may be used as long as it is usually allowed to be added to pharmaceuticals. These tablets and granules can be suitably coated with sugar (sugar coating), gelatin coating, and others as required. If necessary, preservatives, antioxidants and the like may be added thereto.

The pharmaceutical composition of the present disclosure can be prepared into any formulation conventionally produced in the art, and the form of the formulation is not particularly limited but may be preferably an external preparation. The external preparation of the present disclosure may include a conventional form of the external preparation such as a sheet, a liquid coating agent, a spray agent, a lotion agent, a cream agent, a pap agent, a powder agent, a penetration pad agent, a spray agent, a gel agent, a paste agent, a liniment agent, an ointment agent, an aerosol agent, a powder agent, a suspension agent and a percutaneous absorbent. These formations are described in the literature as a prescription commonly known in all pharmaceutical chemistries.

The pharmaceutically effective dose of the present disclosure may vary depending on the kind of wound, application site, treatment frequency, treatment time, formulation, the condition of patients, kind of adjuvant and the like. The dose is not particularly limited, but usually, it may be 0.00001 µg to 10,000 µg when a daily effective dose of the pharmaceutical composition of the present disclosure is applied to a patient. The daily dose may be administered once a day or two or three times a day at appropriate intervals, or intermittently administered at intervals of several days.

However, it is construed that the scope of the present disclosure is not limited by the effective dose in any aspect because the dose of the pharmaceutical composition of the present disclosure may be determined in term of several related factors such as administration route, age, sex, weight, severity of the patient, type of wound, application site, treatment frequency, treatment time, formulation, condition of patients and the kind of adjuvant.

Further, the present disclosure provides the use of inflammatory stimulated mesenchymal stem cells for the prevention or treatment of immune diseases or inflammatory diseases.

Further, the present disclosure provides the use of inflammatory stimulated mesenchymal stem cells for preparing an agent for the prevention or treatment of immune diseases or inflammatory diseases.

Further, the present disclosure provides a method of inhibiting an immune response or an inflammatory response of an individual, the method including the step of administering the inflammatory stimulated mesenchymal stem cells to an individual.

Further, the present disclosure provides a method of preventing or treating an inflammatory disease of an individual, the method including the step of administering the inflammatory stimulated mesenchymal stem cells to an individual.

In the present disclosure, the term "individual" means a mammal including, but not limited to, a cow, a dog, a pig, a chicken, sheep, a horse and a human.

In the present method, the mesenchymal stem cells may be separated by a subfractionation culturing method and may have the property of expressing CD29, CD44, CD49f, CD73, CD90, CD105, CD146, HLA-class I (HLA- I) and Oct4.

The inflammatory stimulated mesenchymal stem cells used in the present method have properties in which they are ones cultured under at least one culture condition selected from the group consisting of mixed lymphocyte reaction (MLR), mitosis-promoting substance (mitogen) treatment and cytokine treatment conditions. The mitosis-promoting substance may include at least one selected from the group consisting of phytohemagglutinin (PHA), concanavalin A (Con A), pokeweed mitogen (PWM), lipopolysaccharide, streptolysin S, a mercury compound and an anti-lymphocyte antibody. The inflammatory stimulated mesenchymal stem cells may be characterized as having a cholinergic neuron-like phenotype.

The inflammatory stimulated mesenchymal stem cells may be characterized by secreting acetylcholine, and the immune diseases or inflammatory diseases may include at least one selected from the group consisting of autoimmune diseases, transplant rejection, arthritis, graft-versus-host-disease, bacterial infection, sepsis and inflammation.

Further, the present disclosure provides a method of preparing mesenchymal stem cells for the prevention or treatment of an immune disease or an inflammatory disease, the method including culturing the mesenchymal stem cells with an inflammatory stimulation.

The inflammatory stimulation includes at least one selected from the group consisting of mixed lymphocyte reaction (MLR) condition, mitosis-promoting substance (mitogen) treatment and cytokine treatment. The mitosis-promoting substance includes at least one selected from the group consisting of phytohemagglutinin (PHA), concanavalin A (Con A), pokeweed mitogen (PWM), lipopolysaccharide, streptolysin S, a mercury compound and an anti-lymphocyte antibody, preferably mixed lymphocyte reaction condition or phytohemagglutinin treatment, but is not limited thereto.

The mesenchymal stem cells produced by the above method release acetylcholine and can be used as immunosuppressants or inflammatory agents.

In the present disclosure, the term "immunosuppressant" refers to an agent including a mesenchymal stem cell cultured in a mixed lymphocyte reaction (MLR) condition, a phytohemagglutinin (PHA) treatment or a cytokine treatment condition or a culture thereof, which can inhibit the immune response to treat immune disorders.

In the present disclosure, the term "anti-inflammatory agent" refers to an agent including a mesenchymal stem cell cultured in a mixed lymphocyte reaction (MLR) condition, a phytohemagglutinin (PHA) treatment or a cytokine treatment condition or a culture thereof, which can inhibit the inflammatory response to treat inflammatory diseases.

In the present disclosure, the term "culturing" means that the mesenchymal stem cells of the present disclosure are grown under moderately artificially controlled environmental conditions.

The mesenchymal stem cells of the present disclosure can be cultured in a conventional medium. The mesenchymal stem cells of the present disclosure include nutrients required for an object to be cultured, that is, cells to be cultured, and additional materials may be added and mixed for special purposes. The medium is also referred to as an incubator or a culture medium and is a concept including all natural medium, synthetic medium and selective medium.

The medium used for culturing should meet the requirements of a specific strain in a suitable manner while controlling temperature, pH, etc. in a conventional medium including a suitable carbon source, nitrogen source, amino acid, vitamin, and the like. The carbon sources that can be used include mixed sugar with glucose and xylose as main carbon sources. A sugar and a carbohydrate such as sucrose, lactose, fructose, maltose, starch and cellulose, an oil such as a soybean oil, a sunflower oil, a castor oil, and a coconut oil, a fatty acid such as fat, palmitic acid, stearic acid, and linoleic acid, an alcohol such as glycerol and ethanol, and organic acids such as acetic acid are additionally included. These materials may be used individually or as a mixture. The nitrogen sources that can be used may include inorganic nitrogen sources such as ammonia, ammonium sulfate, ammonium chloride, ammonium acetate, ammonium phosphate, ammonium carbonate and ammonium nitrate and organic nitrogen sources such as amino acid such as glutamic acid, methionine, and glutamine, peptone, NZ-amine, a meat extract, a yeast extract, a malt extract, a corn steep liquor, casein hydrolyzate, fish or decomposition products thereof, defatted soybean cake or decomposition products thereof. These nitrogen sources may be used alone or in combination. The medium may include potassium (I) phosphate, potassium (II) phosphate, and the corresponding sodium-containing salts as a phosphate source. The phosphate source which may be used includes potassium dihydrogen phosphate or dipotassium hydrogen phosphate or the corresponding sodium-containing salts. Sodium chloride, calcium chloride, iron chloride, magnesium sulfate, iron sulfate, manganese sulfate, calcium carbonate, and the like may be used as the inorganic compound. Finally, essential growth materials such as amino acids and vitamins can be used in addition to these materials.

Further, suitable precursors may be used in the culture medium. The raw materials may be added to the culture in the process of the culture by an appropriate method such as a batch process, a fed-batch process, and a continuous process, but the present disclosure is not particularly limited thereto. Basic compounds such as sodium hydroxide, potassium hydroxide, and ammonia, or acid compounds such as phosphoric acid and sulfuric acid can be used in a suitable manner to adjust the pH of the culture.

Hereinafter, the embodiments are only intended to describe the present disclosure more specifically. Thus, it will be apparent to those skilled in the art that the scope of the present disclosure is not limited by these embodiments in accordance with the concept of the present disclosure.

### Experimental Example 1. Materials and methods

### 1-1. Isolation, Characterization and Culture of Mesenchymal Stem Cells (MSCs)

MSCs are mesenchymal stem cells isolated by the subfractionation culturing method disclosed in Korean Patent No. 10-0802011. The isolation and characteristics of the MSCs were confirmed by cell shape, marker expression and mesenchymal differentiation, and the results are illustrated in FIG. 1. Anti-CD14 (BD Biosciences, San Diego, CA, USA), anti-CD29 (Serotec, Kidlington, UK), anti-CD34 (BD Biosciences), anti-CD44 (Serotec), anti-CD45 (BD Biosciences), anti-CD49f (BD Biosciences), anti-CD73 (BD Biosciences), anti-CD90 (BD Biosciences), anti-CD105 (Serotec), anti-CD106 (BD Biosciences), anti-CD146 (BD Biosciences), anti-HLA class I (BD Biosciences), anti-HLA-DR (BD Biosciences) and anti-Oct4 (BD Biosciences) antibodies were used for flow cytometry. Isotype-matched control antibodies were used as controls. The differentiation possibility of mesenchymal stem cells including adipocyte formation, osteogenesis formation and cartilage differentiation was evaluated with reference to the conventional document (Jung, K. H. et al., Gastroenterology 140, 998-1008 (2011)). MSCs were cultured in CO₂ incubator humidified at 37°C with Dulbecco's modified Eagle's medium containing low glucose (Gibco-BRL, Gaithersburg, MD, USA) supplemented with 10% fetal bovine serum (Gibco-BRL), 1% penicillin/streptomycin (Gibco-BRL) and 1% mycoton (Genlantis, San Diego, CA, USA). When the cells reached 70% to 80%, they were dissociated using with trypsin/EDTA and then sub-cultured for further cultivation.

### 1-2. Immunosuppression assay and inflammatory conditions

Human peripheral blood mononuclear cells (PBMCs) were isolated from two patients, and 1 × 10⁵ PBMCs of each patient were co-cultured in a 96-well plate for the mixed lymphocyte reaction (MLR). To evaluate the effect of the MSCs on lymphocyte proliferation, 4 × 10⁴ MSCs were co-cultured in the MLR for 5 days. For mitogen activation, 2 × 10⁵ PBMCs were stimulated with 1 µg/mL phytohemagglutinin (PHA) (Sigma, St. Louis, MO, USA). Further, 4 × 10⁴ MSCs were co-cultured with the PBMCs for 3 days. As lymphocyte proliferation, the radioactivity emitted by the incorporation of [³H]thymidine (1 µCi/well) was analyzed using a β-counter (Perkin-Elmer, Waltham, MA, USA). The lymphocyte activation by an MLR or PHA treatment was used to confirm inflammatory response.

### 1-3. ELISA

The medium was harvested from nAChR antagonist or agonist-containing co-culture experiments. The secreted proinflammatory cytokines were quantified using the ELISA kits for TNF-α (BD Biosciences; cat. # 555212) and IFN-γ (BD Biosciences; cat. # 555141). The medium in which MLR- or PHA-activated PBMCs were cultured was used to carry out the assay for NGF (R&D Systems, Minneapolis, MN, USA; cat. # DY256-05) and BDNF (R&D Systems; cat. # DBD00).

### 1-4. Isolation of RNA and Performance of semi-quantitative RT-PCR and quantitative RT-PCR (qRT-PCR)

Total RNA was extracted using the easyBlue RNA isolation reagent (Intron, Sungnam, Korea). cDNA was synthesized from 1 g of the total RNA using the AccuPower cDNA Synthesis Kit (Bioneer, Daejeon, Korea). Semi-quantitative RT-PCR was performed using the AccuPower PCR premix (Bioneer). The amplicons were subjected to electrophoresis in 1% agarose gels containing SyberSafe (Invitrogen, Carlsbad, CA, USA) and analyzed using the fluorescence image analyzer LAS4000 mini (Fuji PhotoFilm, Tokyo, Japan). The PCR primer sequences are shown in the following Table 1. The qRT-PCR was performed using primers which were purchased from Applied Biosystems (Foster City, CA, USA) for amplifying TrkA (Hs01021011), TrkB (Hs00178811), TrkC (Hs00176797), p75^{NTR} (Hs00609977), nAChRα7 (Hs04189909), nAChRα5 (Hs00181248), ChAT (Hs00252848), and 18s rRNA (Hs03928985). The qRT-PCR was conducted on a real-time thermal cycler (StepOne Real-Time RT-PCR system, Applied Biosystems).

**[Table 1]**

| Gene | Primers | Sequence (5'-3') | Ta (°C) | Cycle | Products (bp) | SEQ. ID. NOs. |
|---|---|---|---|---|---|---|
| *MAP2* | Forward | TAAGTTTGGAGCAAGCAGTTACAG | 56 | 35 | 508 | 1 |
| | Reverse | TTCTCTCCATACACTTTTGGATCA | | | | 2 |
| *Tuj1* | Forward | AACGAGGCCTCTTCTCACAA | 56 | 25 | 537 | 3 |
| | Reverse | CGATACCAGGTGGTTGAGGT | | | | 4 |
| *GFAP* | Forward | GAGTACCAGTACCTGAAGA | 55 | 30 | 203 | 5 |
| | Reverse | TTCACCACGATGTTCCTCTT | | | | 6 |
| *NF-M* | Forward | TTTGGTTTCCTCTATGATCTCCTC | 54 | 25 | 212 | 7 |
| | Reverse | AGATGGCTCTGGATATAGAAATCG | | | | 8 |
| *nestin* | Forward | TCCAGAGCTGTCAATGACTCTAAG | 56 | 37 | 596 | 9 |
| | Reverse | GACCACTCCAGTTTAGAGGCTAAG | | | | 10 |
| *TrkA* | Forward | GAAGAGTGGTCTCCGTTTCG | 62 | 35 | 410 | 11 |
| | Reverse | CTGACTGCTCCAGCTCTGTG | | | | 12 |
| *TrkB* | Forward | ATCCCTTCCACAGACGTCAC | 50 | 40 | 494 | 13 |
| | Reverse | TCCTGCTCAGGACAGAGGTT | | | | 14 |
| *TrkC* | Forward | ACAAGATGCTTGTGGCTGTG | 201 | 40 | 201 | 15 |
| | Reverse | GGGCCCTGAGGAACTTATTC | | | | 16 |
| p75^{NTR} | Forward | AGCCTTCAAGAGGTGGAACA | 62 | 35 | 447 | 17 |
| | Reverse | CTGCACAGACTCTCCACGAG | | | | 18 |
| *NGF* | Forward | ATACAGGCGGAACCACACTC | 56 | 30 | 408 | 19 |
| | Reverse | GTCTGTGGCGGTGGTCTTAT | | | | 20 |
| *BDNF* | Forward | TGGCTGACACTTTCGAACAC | 54 | 30 | 520 | 21 |
| | Reverse | CTTATGAATCGCCAGCCAAT | | | | 22 |
| *nAchRa3* | Forward | CCATGTCTCAGCTGGTG | 53.5 | 35 | 502 | 23 |
| | Reverse | GTCCTTGAGGTTCATGGA | | | | 24 |
| *nAchRa5* | Forward | GATAATGCAGATGGACGT | 54 | 35 | 506 | 25 |
| | Reverse | TGATGGTATGATCTCTTC | | | | 26 |
| *nAchRa7* | Forward | CCCGGCAAGAGGAGTGAAAGGT | 61 | 31 | 442 | 27 |
| | Reverse | CCGGGCCTCTTCATTCGCAG | | | | 28 |
| *nAchRa9* | Forward | CTACAATGGCAATCAGGTGG | 60 | 30 | 425 | 29 |
| | Reverse | ATGATGGTCAACGCAGTGG | | | | 30 |
| *nAchRb2* | Forward | CAGCTCATCAGTGTGCA | 58.5 | 40 | 410 | 31 |
| | Reverse | GTGCGGTCGTAGGTCCA | | | | 32 |
| *GAPDH* | Forward | GTCATCCATGACAACTTTGGTATC | 56 | 25 | 476 | 33 |
| | Reverse | CTGTAGCCAAATTCGTTGTCATAC | | | | 34 |

### 1-5. Flow cytometry

Anti-GFAP (BD Biosciences; 51449), anti-Tujl (BD Biosciences; 560381), and anti-nestin (BD Biosciences; 56130) antibodies were used for flow cytometry. Isotype-matched control antibodies served as controls. The cells were analyzed using a FACS Calibur flow cytometer (BD Biosciences).

### 1-6. Western blot analysis

The cells for each experimental group were washed twice with PBS and lysed in lysis buffer (50 mM Tris-HCl, 150 mM NaCl, 1 mM ethylenediaminetetraacetic acid (Sigma), 1 mM sodium orthovanadate (Sigma), 1 mM sodium fluoride, 1 mM phenylmethanesulfonylfluoride (Sigma), 1% Triton-X 100, a protease inhibitor cocktail (Pierce, Rockford, IL, USA) and a phosphatase inhibitor cocktail (Pierce)) for 30 minutes on ice. Cell debris was removed by centrifugation at 15,000 × g for 15 minutes, and the supernatants were transferred to a new micro tube. Protein concentration in the cell lysate was measured using the BCA protein assay reagent kit (Pierce). Equal amounts of protein were separated using 10% SDS-polyacrylamide gel electrophoresis under reducing conditions and electro-transferred to Immobilon P membranes (EMD Millipore). For immunodetection, an anti-TrkA (EMD Millipore; cat. # 06-574), anti-TrkC (Cell Signaling Technology, Danvers, MA, USA; cat. # 3376), anti-p75^{NTR} (Cell Signaling Technology; cat. # 8238), anti-ChAT (EMD Millipore; cat. # AB144P), anti-nAChR α7 (Alomone, Jerusalem, Israel; cat. # ANC-007), or anti-Actin (Santa Cruz Biotechnology, Dallas, TX, USA; cat. # SC-47778) antibodies were used as a primary antibody. After incubation with secondary antibody conjugated with horseradish peroxidase, the enhanced chemoluminescence of bands was detected using West-Zol Plus (Intron).

### 1-8. ACh assay

MSCs stimulated with MLR or PHA were incubated for 24 hours, and the respective MSCs were harvested. ACh/choline was using the Enzychrom Acetylcholine Assay Kit (BioAssay Systems, Hayward, CA, USA; EACL-100), according to the manufacturer's instructions.

### 1-9. Confirmation of spheroid formation

Prior to cell inoculating, a petri dish (diameter, 100 mm) was coated with 1% Pluronic F-127 (Sigma; dissolved in distilled water) for 30 minutes at room temperature, and then it was thoroughly washed with phosphate-buffered saline (PBS). A total of 1 × 10⁶ MSCs were seeded onto the dish containing 1% non-essential amino acids (Gibco-BRL), 1% L-glutamine (Gibco-BRL), 1% N2 supplement (Gibco-BRL), 20 ng/ml epidermal growth factor (R&D Systems), and 20 ng/ml basic fibroblast growth factor (R&D Systems) and then were incubated for 24 hours to 72 hours.

### 1-10. Immunofluorescence staining and use of confocal microscopy-

The cells for each experimental group were fixed with 4% paraformaldehyde and permeabilized using 0.5% TritonX-100 (Sigma) dissolved in PBS. The cells were labeled with primary antibodies (1 : 200 to 1 : 1000 dilution) such as an anti-TrkA (EMD Millipore, Billerica, MA, USA; cat. # 06-574), anti-ChAT (EMD Millipore; cat. # AB144P), anti-Tujl (EMD Millipore; cat. # MAB1637), anti-TH (Cell Signaling Technology; cat. # 2792), anti-NCAM1 (EMD Millipore; cat. # CBL275), anti-MBP (EMD Millipore; cat. # AB980), anti-O4 (Sigma; cat. # 07139), anti-NF-M (EMD Millipore; cat. # AB1987), anti-Nestin (EMD Millipore; cat. # MAB5326), or anti-GABA (Abcam, Cambridge, MA, USA; cat. # Ab86186) antibodies overnight at 4°C. After incubation with the primary antibodies, the cells were incubated for 1 hour with an AlexaFluor488 or AlexaFluor594-conjugated secondary antibody (Molecular Probes, Carlsbad, CA, USA) (1 : 300 dilution). The cells were stained with 4,6-diamidino-2-phenylindole (DAPI; molecular probes) for 1 minute. They were observed using confocal microscope (Zeiss LSM510 Meta Confocal Imaging System; Carl Zeiss, Thornwood, NY, USA).

### 1-11. Treatment with a cholinergic antagonist or agonist

α-Bungarotoxin (EMD Millipore; cat. # 203980), an nAChRα7 antagonist, was added to the medium at the concentration of 1 M. As cholinergic agonists, ACh chloride (Sigma; cat. # A6625) and carbachol (EMD Millipore; cat. # 212385) were used at the concentrations of 1 nM and 10 pM, respectively, and the experiments were conducted.

### Example 1. Characterization of the MSCs of the present disclosure

MSCs of the present disclosure were separated by a subfractionation culturing method. The MSCs of the present disclosure have advantages in which they are not mixed with other types of cells except stem cells, and only stem cells are isolated to have the high purity compared to the MSCs isolated by the conventional density gradient centrifugation method. The MSCs were used to confirm the properties of MSCs which were used in the following series of experiments.

In particular, after the MSCs of the present disclosure were cultured, their morphological characteristics were confirmed. In order to further identify the differentiation potential of MSCs, visualization was performed using Oil Red O, an adipocyte-specific lipid droplet, visualization was performed using Safranin O, a cartilage-specific proteoglycan, and the bone-specific mineralization was visualized using Alizarin Red S. Further, in order to confirm the properties of MSCs of the present disclosure, the flow cytometry was performed using positive markers CD29, CD44, CD49f, CD73, CD90, CD105, CD146, HLA-class I (HLA-I) and Oct4. The negative markers used were hematopoietic/endothelial markers including CD14, CD34, CD45, CD106 and HLA-DR. The results are illustrated in FIG. 1.

As illustrated in FIG. 1A, it was confirmed that the MSCs of the present disclosure manifested fibroblast-like morphology.

As illustrated in FIG. 1B, it was confirmed that the MSCs of the present disclosure had adipogenically, chondrogenically and osteogenically differentiation potentials.

As illustrated in FIG. 1C, it was confirmed that positive markers CD29, CD44, CD49f, CD73, CD90, CD105, CD146, HLA-class I (HLA-I) and Oct4 were normally expressed in the MSCs of the present disclosure.

### Example 2. Confirmation of Inhibitory effect of MSCs on lymphocyte proliferation under inflammatory conditions, morphological changes, spheroid entity formation and relationship with immunosuppressants

### 2-1. Confirmation of Inhibitory effect of MSCs on lymphocyte proliferation under inflammatory conditions and morphological changes

Lymphocyte proliferation inhibitory effect and morphological changes of human MSCs under inflammatory conditions (mixed lymphocyte reaction (MLR) condition or phytohemagglutinin (PHA) treatment condition) were confirmed.

Specifically, according to the method described in Experimental Example 1-2, the mixed lymphocyte reaction (MLR) condition was set such that PBMCs obtained from different donors were cultured together, and the phytohemagglutinin (PHA) treatment condition was set such that PBMCs were treated with PHA. The inhibitory effects and morphological changes of lymphocyte proliferation by alloantigenic immune responses were confirmed for each PBMCs only group (P and Po) obtained from different donors; MSCs group co-cultured in mixed lymphocyte reaction (MLR) condition (MSC in MLR); mixed lymphocyte reaction condition group without MSCs (MLR); or MSCs group co-cultured in phytohemagglutinin (PHA) treatment condition (MSC in P^{PHA}) according to inflammatory conditions. The results are illustrated in FIG. 2.

As illustrated in FIG. 2A, it was confirmed that the MSCs co-cultured in mixed lymphocyte reaction (MLR) condition effectively inhibited lymphocyte proliferation induced by alloantigenic immune response.

As illustrated in FIG. 2B, it was confirmed that the MSCs co-cultured in phytohemagglutinin (PHA) condition inhibited lymphocyte proliferation.

As illustrated in FIG. 2C, it was confirmed that the MSCs co-cultured in mixed lymphocyte reaction (MLR) condition or phytohemagglutinin (PHA) condition showed abnormal morphological changes including remarkable elongation, filamentation and branching.

As illustrated in FIGS. 2D and 2E, it was confirmed that the MSCs co-cultured in mixed lymphocyte reaction (MLR) condition or phytohemagglutinin (PHA) condition showed morphological features similar to nerve cells such as dendritic-like cells. Further, it was confirmed that cells connected to neighboring cells via the dendrite-like paths.

As illustrated in FIGS. 2F and 2G, it was confirmed that MSCs showed neuron-like morphology under the inflammatory conditions.

Thus, it was confirmed that MSCs had abnormal morphological changes in inflammatory conditions (mixed lymphocyte reaction (MLR) conditions or phytohemagglutinin (PHA) treatment conditions).

### 2-2. Spheroid entity formation from MSCs under inflammatory conditions and relationship with immunosuppression

It was confirmed whether or not the spheroid entities were formed from MSCs for inflammatory conditions and the death of the entities was more related to immunosuppression than death due to anoikis mechanism of action.

Specifically, according to the method described in Experimental Example 1-2, the mixed lymphocyte reaction (MLR) condition was set such that PBMCs obtained from different donors were cultured together, and the phytohemagglutinin (PHA) treatment condition was set such that PBMCs were treated with PHA. Then, it was confirmed whether or not the spheroid entities were formed. Further, in order to minimize and neutralize the effect of the mixed lymphocyte reaction (MLR) condition, the supernatant containing the structure of spheroid entities without MSCs was transferred to a fresh medium and cultured for 24 hours. The results are illustrated in FIG. 3.

As illustrated in FIG. 3A, it was confirmed that spheroid entities were produced in MSCs by the inflammatory condition of MLR.

As illustrated in FIG. 3B, it was confirmed that when the inflammatory condition of MLR was minimized, MSCs showed fibroblast-like MSCs morphology attached to a medium other than spheroid entities.

Thus, it was confirmed that when the inflammatory conditions were minimized, the formation of spheroid entities was inhibited, and fibroblast-like MSCs were expressed so that the inflammatory conditions induced the generation of spheroid entities from MSCs and the change in properties of MSCs.

### Example 3. Confirmation of neuron-like properties of MSCs under inflammatory conditions

It was confirmed that human MSCs were changed to have neuron-like properties under inflammatory conditions (mixed lymphocyte reaction (MLR) conditions or phytohemagglutinin (PHA) treatment conditions).

Specifically, it was confirmed that the nerve growth factor receptor was expressed according to the method described in Experimental Example 1-2. The mixed lymphocyte reaction (MLR) condition was set such that PBMCs obtained from different donors were cultured together, and the phytohemagglutinin (PHA) treatment condition was set such that PBMCs were treated with PHA. Semi-quantitative RT-PCR was performed according to the method of Experimental Example 1-4 to confirm the expression of nestin, Tuj1, MAP2, NF-M and GFAP for each PBMCs only group (P and Po) obtained from different donors; MSCs group co-cultured in mixed lymphocyte reaction (MLR) condition (MSC in MLR); mixed lymphocyte reaction condition group without MSCs (MLR); or MSCs group co-cultured in phytohemagglutinin (PHA) treatment condition (MSC in P^{PHA}) according to inflammatory conditions. Further, the flow cytometry analysis of nestin, Tuj1 and GFAP was performed according to the method of Experimental Examples 1-5. Further, the expression levels of nestin, Tuj1, NCAM1, GFAP and 04 were confirmed by immunofluorescence staining according to the method of Experimental Example 1-9. The results are illustrated in FIG. 4.

As illustrated in FIG. 4A, it was confirmed that neuroglial markers such as Tuj1 and MAP2 were expressed, but astrocytes (astrocytic cell) such as GFAP was not expressed in MSCs cultured under inflammatory conditions (mixed lymphocyte reaction (MLR) condition or phytohemagglutinin (PHA) treatment condition).

As illustrated in FIG. 4B, it was confirmed that nestin and Tuj1 were expressed, but GFAP was not expressed in MSCs cultured under inflammatory conditions (mixed lymphocyte reaction (MLR) condition or phytohemagglutinin (PHA) treatment condition).

As illustrated in FIGS. 4C and 4D, it was confirmed that neuroglial markers such as nestin and Tuj1 were expressed in MSCs cultured under inflammatory conditions (mixed lymphocyte reaction (MLR) condition or phytohemagglutinin (PHA) treatment condition). However, it was confirmed that glial markers including astrocyte (astrocytic cell) markers such as GFAP and oligodendrocyte markers such as 04 were not expressed.

Thus, it was confirmed that the MSCs cultured under inflammatory conditions (mixed lymphocyte reaction (MLR) condition or phytohemagglutinin (PHA) treatment condition) inhibit imperfect and immature neuronal cell characteristics and exhibit similar characteristics to neuronal cells.

### Example 4. Confirmation of nerve growth factor receptor (neurotrophin receptor) and nerve growth factor expression of MSCs under inflammatory condition

### 4-1. Confirmation of nerve growth factor receptor expression of MSCs under inflammatory condition

In order to confirm the expression of the nerve growth factor receptor of MSCs under the inflammatory condition, expression of nerve growth factor receptor was confirmed by the method described in Experimental Example 1-2. The mixed lymphocyte reaction (MLR) condition is that each PBMCs obtained from different donors were co-cultured and PHA treatment condition is that PHA was treated to PBMCs. The expression of nerve growth factor receptors including Trk family (A, B and C) and p75^{NTR} was confirmed for each PBMCs only group (P and Po) obtained from different donors; MSCs group co-cultured in mixed lymphocyte reaction (MLR) condition (MSC in MLR); mixed lymphocyte reaction condition group without MSCs (MLR); or MSCs group co-cultured in phytohemagglutinin (PHA) treatment condition (MSC in P^{PHA}); or PHA treatment group to normal MSC (MSC + PHA) according to inflammatory conditions.

According to the method described in Experimental Example 1-4, semi-quantitative RT-PCR or qRT-PCR was performed to confirm the expressions of TrkA, TrkB, TrkC and p75^{NTR}. Further, according to the method described in Experimental Example 1-6, western blotting was performed to confirm the expression of TrkA, TrkB, TrkC and p75^{NTR}. Further, according to the method described in Experimental Example 1-9, immunofluorescence staining was performed to confirm the expression level of TrkA. The results are illustrated in FIG. 5.

As illustrated in FIGS. 5A, 5B and 5C, it was confirmed that TrkA and p75^{NTR} were significantly expressed, but TrkB and TrkC were poorly expressed in MSCs cultured under inflammatory conditions (mixed lymphocyte reaction (MLR) condition or phytohemagglutinin (PHA) treatment condition).

As illustrated in FIGS. 5E and 5E, it was confirmed that TrkA proteins were expressed in MSCs cultured under inflammatory conditions (mixed lymphocyte reaction (MLR) condition or phytohemagglutinin (PHA) treatment condition).

### 4-2. Confirmation of nerve growth factor expression in PBMCs under inflammatory conditions

Expression of nerve growth factor (NGF) and brain-derived growth factor (BDNF), known as a nerve growth factor, was confirmed in PBMCs.

Specifically, according to the method described in Example 3-1, semi-quantitative RT-PCR or qRT-PCR was performed to confirm the expression of NGF and BDNF. Further, according to the method described in Experimental Example 1-3, ELISA was performed to confirm the expression of NGF and BDNF. The results are illustrated in FIG. 6.

As illustrated in FIGS. 6A to 6H, it was confirmed that expression levels of NGF and BDNF were significantly increased in the PBMCs cultured under inflammatory conditions (mixed lymphocyte reaction (MLR) condition or phytohemagglutinin (PHA) treatment condition).

Thus, it was confirmed that both nerve growth factor receptor and nerve growth factor were expressed in the PBMCs cultured under inflammatory conditions (mixed lymphocyte reaction (MLR) condition or phytohemagglutinin (PHA) treatment condition) so that the MSCs were stimulated to show neuron-like properties.

### Example 5. Confirmation of inhibitory effect of neurosphere-like spheroids constituting MSCs on neuronal marker expression and lymphocyte proliferation under inflammatory conditions

The inhibitory effect of neurosphere-like spheroids constituting MSCs of neuronal marker expression and lymphocyte proliferation was confirmed under inflammatory conditions.

### 5-1. Confirmation of inhibitory effect of neurosphere-like spheroids constituting MSCs on neuronal marker expression under inflammatory conditions

Specifically, in order to confirm whether the spheroidal structure in MSCs under the inflammatory conditions as illustrated in Example 1 is similar to that of neurospheres and the neurosphere's property of neurosphere-like spheroids constituting MSCs under the inflammatory conditions of the present disclosure, the spheroids of MSCs under the inflammatory conditions were obtained according to the method described in Experimental Example 1-8, placed in a Matrigel-coated medium and cultured on a medium. Then, the spheroids attached to the medium were identified. Further, in order to confirm nerve cell marker expression of the spheroids, MSCs were cultured in the neurogenic differentiation medium containing neurobasal medium (Gibco-BRL), 2% B27 supplement (Gibco-BRL), 1% non-essential amino acid, 20 ng/ml epithelial cell growth factor, 40 ng/ml basic fibroblast growth factor and 10 ng/ml fibroblast growth factor 8 (Peprotech, Rehovot, Israel). Then, the MSCs were immunofluorescently stained to confirm the expression of nerve cell markers such as Tuj1, NF-M and MAP2 and the expression of neural progenitor cell (NPC) markers such as nestin. The results are illustrated in FIG. 7.

As illustrated in FIG. 7A, it was confirmed that the MSCs under the inflammatory conditions successfully produced spheroids. Further, as illustrated in FIG. 7B, it was confirmed that nerve cell markers such as Tuj1, NF-M and MAP2 were expressed, and neural progenitor cell (NPC) markers such as nestin were expressed, thereby showing the neurosphere's property of neurosphere-like spheroids constituting MSCs under the inflammatory conditions of the present disclosure.

### 5-2. Confirmation of the inhibitory effect of neurosphere-like spheroids constituting MSCs on lymphocyte proliferation under inflammatory conditions

In order to confirm the relationship between PBMCs and neurosphere-like spheroids constituting MSCs under the inflammatory conditions, the experiment was conducted such that the PBMCs obtained from different donors were cultured together under the mixed lymphocyte reaction (MLR) condition, and the experiment was conducted such that the PBMCs treated with PHA were cultured for 3 days to 5 days under the phytohemagglutinin (PHA) treatment condition, thereby confirming the formation of spheroids.

In order to confirm whether MSC spheroids inhibit lymphocyte proliferation under the MLR inflammatory conditions, it was compared with the MSCs cultured with a single membrane-dependent cell count. Specifically, the inhibitory levels of lymphocyte proliferation were confirmed for each PBMCs only group obtained from different donors (P or Po); mixed lymphocyte reaction condition group without MSCs (MLR); phytohemagglutinin condition group without MSCs (PHA); groups cultured at a ratio of 1 : 2, 1 : 5, and 1 : 20 between MSCs spheroids and MLR or PHA-activated PBMCs (spheroids); groups cultured at a ratio of 1 : 2, 1 : 5, and 1 : 20 between MSCs monolayer and MLR or PHA-activated PBMCs (monolayer).

Further, as illustrated in Experimental Example 1-5, the flow cytometry was performed to determine the expression levels of Tuj 1 and nestin of MSCs spheroids under the inflammatory conditions (mixed lymphocyte reaction (MLR) condition or phytohemagglutinin (PHA) treatment condition). The results are illustrated in FIG. 8.

As illustrated in FIG. 8A, it was confirmed that spheroidal spheroids were formed in PBMCs (MLR) activated under the inflammatory condition, and thus a correlation between PBMCs and MSC spheroids was formed.

As illustrated in FIGS. 8B and 8C, it was confirmed that the higher the ratio of MSCs steroids, the more effective the inhibition of lymphocyte proliferation in the mixed culture groups of MLR- or PHA-activated PBMCs and MSCs spheroids (spheroid). Further, it was confirmed that the lymphocyte proliferation inhibition was further activated in the mixed culture group of MLR- or PHA-activated PBMCs and MSCs monolayers (monolayer).

As illustrated in FIG. 8D, it was confirmed that the expression of Tuj1 and nestin of MSCs spheroids was increased in the inflammatory conditions (mixed lymphocyte reaction (MLR) condition or phytohemagglutinin (PHA) treatment condition).

### 5-3. Confirmation of nerve cell marker expression of neurosphere-like spheroids in MSCs under the inflammatory conditions according to high neurogenic potential

In order to confirm nerve cell marker expression of neurosphere-like spheroids in MSCs under the inflammatory conditions according to high neurogenic potential, MSCs without any treatment were cultured in a neurobasal medium containing 2% N₂ supplement (Gibco-BRL), 1% non-essential amino acids, 10 ng/ml BDNF, 20 ng/ml epithelial growth factor, 40 ng/ml basic fibroblast growth factor and 10 ng/ml fibroblast growth factor 8 as control groups. They were referred to as MSC1 and MSC2, respectively. Then, it was confirmed that nerve cell markers or neural precursor cell markers such as Tuj1, nestin, MBP and NF-M were expressed.

Further, each PBMCs obtained from different donors were co-cultured with MSC under the mixed lymphocyte reaction (MLR) condition, and they were cultured in a neurobasal medium containing 2% N₂ supplement (Gibco-BRL), 1% non-essential amino acids, 10 ng/ml BDNF, 20 ng/ml epithelial growth factor, 40 ng/ml basic fibroblast growth factor and 10 ng/ml fibroblast growth factor 8. They were referred to as MSC1 and MSC2, respectively. Then, it was confirmed that nerve cell markers or neural precursor cell markers such as Tuj1, nestin, MBP and NF-M were expressed. The results are illustrated in FIG. 9.

As illustrated in FIGS. 9A and 9B, it was confirmed that the nerve cell markers were expressed in the neurosphere-like spheroids and that the neurosphere-like spheroids can perform similar functions as neuron-like cells.

### Example 6. Confirmation of acetylcholine (ACh) secretion of MSCs under the inflammatory conditions

### 6-1.Confirmation of acetylcholine (ACh) secretion of MSCs under the inflammatory conditions

MSCs under the inflammatory conditions were confirmed to have neuronal-like properties, thereby confirming the secretion of the neurotransmitter such as acetylcholine (ACh).

Specifically, as the methods described in Experimental Examples 1-2 and 1-7, the mixed lymphocyte reaction (MLR) condition refers to a condition in which each PBMCs obtained from different donors were cultured together, and the phytohemagglutinin (PHA) treatment condition refers to a condition in which PBMCs were treated with PHA. The inflammatory condition includes two conditions as described above. According to the method described in Experimental Example 1-5, qRT-PCR was performed to confirm the expression level of ChAT (choline acetyltransferase) for each PBMCs only group (P and Po) obtained from different donors; MSCs group co-cultured in mixed lymphocyte reaction (MLR) condition (MSC in MLR); mixed lymphocyte reaction condition group without MSCs (MLR); or MSCs group co-cultured in phytohemagglutinin (PHA) treatment condition (MSC in P^{PHA}) according to the inflammatory conditions. Further, according to the method described in Experimental Example 1-9, immunofluorescence staining was performed to confirm the expression levels of ChAT, TH (tyrosine hydroxylase) and GABA (γ-aminobutyric acid); or ChAT, NCAM1, MBP, Tuj1, NF-M, nestin, TrkA and GABA. The results are illustrated in FIG. 10.

As illustrated in FIG. 10A, it was confirmed that cholinergic ChAT was expressed, but GABAergic GABA and dopaminergic TH were not expressed in MSCs cultured under inflammatory conditions (mixed lymphocyte reaction (MLR) condition or phytohemagglutinin (PHA) treatment condition).

As illustrated in FIGS. 10B and 10C, it was confirmed that MSCs cultured under inflammatory conditions (mixed lymphocyte reaction (MLR) condition or phytohemagglutinin (PHA) treatment condition) showed higher expression of ChAT than cells in other conditions.

As illustrated in FIG. 10D, it was confirmed that when MSCs spheroids were under the inflammatory conditions (mixed lymphocyte reaction (MLR) condition or phytohemagglutinin (PHA) treatment condition), MSCs were induced to have cholinergic properties.

As illustrated in FIGS. 10E and 10F, it was confirmed that the acetylcholine was secreted by MSCs cultured under the inflammatory conditions (mixed lymphocyte reaction (MLR) condition or phytohemagglutinin (PHA) treatment condition).

### 6-2. Confirmation of change in MSCs to cholinergic neuron-like phenotypes by soluble factors secreted under the inflammatory conditions

An experiment was conducted using a transwell plate to confirm that cell-cell contact under the inflammatory conditions did not induce changes to cholinergic neuron-like phenotypes and that some secreted soluble factors induce a change in MSCs to cholinergic neurons-like phenotypes.

Specifically, 100,000 MSCs were inoculated into the lower wells in a 0.4 m-pore transwell plate (Corning, Tewksbury, MA, USA) and mixed lymphocyte reaction (MLR)-activated PBMCs (1 × 10⁶) (bottom: MSC and insert: P + Po) or medium (bottom: MSC and insert: medium) was inserted, and then the cells were cultured for 3 days. Further, a general culture method other than Transwell was used, and MSCs were cultured under mixed lymphocyte reaction (MLR) conditions (MSC in MLR (co-culture)) to confirm cholinergic neuron-like phenotypes.

MSCs which is not cultured in conditioned medium (CM) (no CM); MSCs cultured in conditioned medium (CM) of activated PBMCs under the mixed lymphocyte reaction (MLR) condition (CM from MLR); and MSCs cultured in conditioned medium (CM) of activated PBMCs under the mixed lymphocyte reaction (MLR) condition (CM from P^{PHA}) were used to confirm changes in MSCs into cholinergic neuron-like phenotypes. The results are illustrated in FIG. 11.

As illustrated in FIG. 11A, Transwell assay was performed to confirm neuro-like morphological changes of MSCs cultured in the inflammatory conditions (mixed lymphocyte reaction (MLR) condition or phytohemagglutinin (PHA) treatment condition).

As illustrated in FIGS. 11B and 11C, it was confirmed that the MSCs exhibited cholinergic neuron-like phenotypes even under conditioned medium (CM) condition and that ChAT, NCAM1, NF-M and Tuj1 were also expressed.

### Example 7. Confirmation of nicotinic cholinergic receptor expression and cholinergic inhibitory mechanisms of PBMCs under the inflammatory conditions

### 7-1. Confirmation of nicotinic cholinergic receptor expression of PBMCs under the inflammatory conditions

In order to confirm whether PBMCs expresses nicotinic cholinergic receptor under the inflammatory conditions, experiments were performed according to the method described in Experimental Example 1-2. The mixed lymphocyte reaction (MLR) condition was set such that PBMCs obtained from different donors were cultured together, and the phytohemagglutinin (PHA) treatment condition was set such that PBMCs were treated with PHA. According to inflammatory conditions, each PBMCs only group (P and Po) obtained from different donors; MSCs group co-cultured in mixed lymphocyte reaction (MLR) condition (MSC in MLR); mixed lymphocyte reaction condition group without MSCs (MLR); or MSCs group co-cultured in phytohemagglutinin (PHA) treatment condition (MSC in P^{PHA}) were placed.

According to the method described in Experimental Example 1-5, semi-quantitative RT-PCR was performed to confirm the expression levels of nAChRα3, nAChRα5, nAChRα7, nAChRα8 and nAChRβ2, and qRT-PCR was performed to confirm the expression levels of nAChRα5 or nAChRα7. Further, according to the method described in Experimental Example 1-6, western blotting was performed to confirm the expression levels of nAChRα7. The results are illustrated in FIG. 12.

As illustrated in FIGS. 12A to 12C, it was confirmed that PBMCs spheroids expressed nAChRα5 and nAChRα7 under the inflammatory conditions (mixed lymphocyte reaction (MLR) condition or phytohemagglutinin (PHA) treatment condition).

As illustrated in FIG. 12D, it was confirmed that PBMCs spheroids expressed nAChRα7 under the inflammatory conditions (mixed lymphocyte reaction (MLR) condition or phytohemagglutinin (PHA) treatment condition).

### 7-2. Confirmation of nicotinic cholinergic receptor expression of MSCs

In order to confirm whether MSCs expresses nicotinic cholinergic receptor, experiments were performed according to the method described in Experimental Example 1-2. MSCs only group (MSC control); MSCs group co-cultured in mixed lymphocyte reaction (MLR) condition (MSC in MLR); or MSCs group co-cultured in phytohemagglutinin (PHA) treatment condition (MSC in P^{PHA}) were placed. The results are illustrated in FIG. 13.

As illustrated in FIG. 13, it was confirmed that the MSCs only group had little or no expression of nAChRα7.

Therefore, according to Examples 7-1 and 7-2, it was confirmed that nAChRα7, nicotinic cholinergic receptor, was expressed in only PBMCs, an immune cell. Accordingly, it was confirmed that acetylcholine secreted from MSC, a stem cell, bound only to the immune cells in which the nicotinic cholinergic receptors were expressed.

### 7-3. Confirmation of ACh-nAChR pathway mechanism of MSCs under the inflammatory conditions

In order to confirm ACh-nAChR pathway mechanism of MSCs under the inflammatory conditions, experiments were performed according to the methods described in Experimental Examples 1-2 and 1-10. The mixed lymphocyte reaction (MLR) condition was set such that PBMCs obtained from different donors were cultured together, and the phytohemagglutinin (PHA) treatment condition was set such that PBMCs were treated with PHA. According to inflammatory conditions, each PBMCs only group (P and Po) obtained from different donors; MSCs group co-cultured in mixed lymphocyte reaction (MLR) condition (MSC in MLR); mixed lymphocyte reaction condition group without MSCs (MLR); or MSCs group co-cultured in phytohemagglutinin (PHA) treatment condition (MSC in P^{PHA}) were placed. A cholinergic antagonist such as α-bungarotoxin (α-BTX, an antagonist of nAChRα7); a cholinergic agonist such as ACh chloride (ACh-Cl, an agonist of AChR); or a non-specific cholinergic agonist such as carbachol was added to the medium, and the activity of TNF-α and IFN-γ of MSCs by each inflammatory condition was confirmed. The results are illustrated in FIG. 14.

As illustrated in FIGS. 14A to 14C, it was confirmed that lymphocyte proliferation was inhibited by MSCs cultured under the mixed lymphocyte reaction (MLR) condition, which is an inflammatory condition, and that TNF-α and IFN-γ were activated again when cultured with α-BTX.

As illustrated in FIGS. 14D to 14F, it was confirmed that lymphocyte proliferation was inhibited by MSCs cultured under the phytohemagglutinin (PHA) treatment condition, which is an inflammatory condition, and that TNF-α and IFN-γ were activated again when cultured with a-BTX.

As illustrated in FIGS. 14G to 14I, it was confirmed that lymphocyte proliferation was inhibited by MSCs cultured under the mixed lymphocyte reaction (MLR) condition, which is an inflammatory condition, and that TNF-α and IFN-γ were significantly inhibited when cultured with ACh-Cl.

As illustrated in FIGS. 14J to 14L, it was confirmed that lymphocyte proliferation was inhibited by MSCs cultured under the phytohemagglutinin (PHA) treatment condition, which is an inflammatory condition, and that TNF-α and IFN-γ were significantly inhibited when cultured with ACh-Cl.

As illustrated in FIGS. 15A to 15D, it was confirmed that when carbachol, a non-specific cholinergic agonist was added to the MSCs culture medium under the inflammatory condition, and then the mixture was cultured, the MSCs inhibited the lymphocyte proliferation and the inflammatory cytokine release so that the ACh-nAChR pathway mechanism was identified in both specific and non-specific cholinergic agonists.

### Example 8. Confirmation of inflammatory stimulation, morphological changes and neuron-like cell properties of MSCs by treatment of mouse or rat with inflammatory cells

### Example 8-1. Confirmation of inflammatory stimulation, morphological changes and neuron-like cell properties of MSCs by treatment of mouse or rat with inflammatory cells

By treatment with splenocytes, one of the inflammation-related cells of the mouse to confirm the inflammatory stimulation of MSCs. Accordingly, the morphological changes of MSCs and the degree of change to the cholinergic neuron-like phenotype were confirmed.

Specifically, splenocytes (SP) of C57BL/6 mouse (Orient, Sungnam, Korea) were cultured with 1 µg/ml anti-CD3 (BD Biosciences) and anti-CD28 (BD Biosciences) antibodies (αCD3/CD28) to result in the activation thereof. Then, splenocytes (2 × 10⁵) of CD3/CD28-activated mouse and MSCs (4 × 10³ to 4 × 10⁴) were cultured at a ratio of 1 : 5, 1 : 10, 1 : 20 and 1 : 50 to confirm their morphological changes.

Further, the cholinergic neurons or neural precursor cell-related markers ChAT, NCAM1, Tuj1, NF-M, nestin, MBP and TH were used to confirm their expression. The results are illustrated in FIG. 16.

As illustrated in FIG. 16A, it was confirmed that MSCs treated with splenocytes of mice did not show inflammatory stimulation, and thus the lymphocyte proliferation was not properly inhibited.

As illustrated in FIG. 16B, it was confirmed that MSCs treated with splenocytes of mice did not show inflammatory stimulation, and thus it was not changed to a cholinergic neuron-like phenotype.

As illustrated in FIG. 16C, it was confirmed that MSCs treated with splenocytes of mice did not show inflammatory stimulation, and thus cholinergic neurons or neural precursor cell-related markers ChAT, NCAM1, Tuj1, NF-M, nestin, MBP and TH were not expressed.

### Example 8-2. Confirmation of inflammatory stimulation, morphological changes and neuron-like cell properties of MSCs by treatment of rat with inflammatory cells

By treatment with splenocytes, one of the inflammation-related cells of the rat to confirm the inflammatory stimulation of MSCs. Accordingly, the morphological changes of MSCs and the degree of change to the cholinergic neuron-like phenotype were confirmed.

Specifically, splenocytes (SP) of Sprague-Dawley rat (Orient, Sungnam, Korea) were cultured with 1 µg/ml anti-CD3 (BD Biosciences) and anti-CD28 (BD Biosciences) antibodies (αCD3/CD28) to result in the activation thereof. Then, splenocytes (2 × 10⁵) of CD3/CD28-activated mouse and MSCs (4 × 10³ to 4 × 10⁴) were cultured at a ratio of 1 : 5, 1 : 10 and 1 : 20 to confirm their morphological changes.

Further, the cholinergic neurons or neural precursor cell-related markers ChAT, NCAM1, Tuj1, NF-M, nestin, MBP, TH and GABA were used to confirm their expression. The mRNA expression levels of TrkA, TrkB, TrkC and p75^{NTR} which are nerve growth factor receptors of MSCs treated with rat's splenocyte were confirmed. The results are illustrated in FIG. 17.

As illustrated in FIG. 17A, it was confirmed that MSCs treated with splenocytes of rat did not show inflammatory stimulation, and thus the lymphocyte proliferation was not properly inhibited.

As illustrated in FIG. 17B, it was confirmed that MSCs treated with splenocytes of rat did not show inflammatory stimulation, and thus it was not changed to a cholinergic neuron-like phenotype.

As illustrated in FIG. 17C, it was confirmed that MSCs treated with splenocytes of rat did not show inflammatory stimulation, and thus cholinergic neurons or neural precursor cell-related markers ChAT, NCAM1, Tuj1, NF-M, nestin, MBP, TH and GABA were not expressed.

As illustrated in FIG. 17D, it was confirmed that MSCs treated with splenocytes of rat did not show inflammatory stimulation, and thus the mRNA expression levels of TrkA, TrkB, TrkC and p75^{NTR} which are nerve growth factor receptors were lower than those of normal MSCs.

Therefore, it was confirmed that changes in MSCs to cholinergic neuron-like phenotype and neuron-like properties are human-specific phenomena that do not occur in mice or rats.

### Example 9. Confirmation of ChAT or nestin expression and MSC cytoplasmic generation of human cells in mouse

ChAT or nestin expression and MSC cytoplasm generation of human cells in the mouse were determined by treatment with PBMCs and MSCs.

Specifically, in order to prepare a transient humanized GVHD mouse model, 10-week-old Balb/c male mice (Central Lab Animal, Seoul, Korea) (n = 3) were irradiated with 8.5 Gy (4MV Linac; Siemens, Berlin, Germany). After 24 hours of irradiation, the mice were injected with PBMCs (PI or P2; 5 × 10⁶ cells/each/head) or mixed PBMCs (PI + P2, 1 × 10⁷ cells/head, mixing ratio = 1 : 1). Thereafter, human MSCs (1 × 10⁶ cells/head) were injected into the mice, respectively. After 48 hours, the mice were sacrificed and secondary lymphoid organs (spleen and superficial cervical, axillary, mesenteric and inguinal lymph nodes) were removed. The removed organs were frozen, and their sections were prepared. In order to confirm ChAT⁺ nestin⁺ human cells, each mouse was immunofluorescently stained with human-specific anti-ChAT and anti-nestin antibodies to confirm them. DAPI was used to confirm nuclear staining. For MSC tracking experiments, each mouse was labeled with biocompatible silica-coated fluorescent nanoparticles (Neostem; Biterials, Seoul, Korea) according to the manufacturer' s protocol in order to confirm the cytoplasm of MSC. The results are illustrated in FIG. 18.

As illustrated in FIG. 18B, it was confirmed that anti-ChAT and anti-nestin antibodies were expressed in mesenteric lymph nodes of mice injected with human MSCs after the injection of mixed PBMCs (P1 + P2).

As illustrated in FIG. 18C, it was confirmed that MSC cytoplasm was found in mice injected with human MSCs after the injection of the mixed PBMCs (PI + P2).

As illustrated in FIG. 19, the above series of experiments confirm that the treatment of inflammatory conditions (mixed lymphocyte reaction (MLR) conditions or phytohemagglutinin (PHA) treatment conditions) of the present disclosure changes MSCs to cholinergic neuron-like phenotype, rather than transdifferentiation to actual neuronal cells. The MSCs treated with inflammatory conditions increased the expression of neurotrophin receptors and nicotinic cholinergic receptors. Specifically, the expression of TrkA and p75^{NTR} in the neurotrophin receptors was significantly increased. The cells were stimulated by nerve growth factor NGF and BDNFs expressed in the immune cells in the inflammatory environment to result in changes into a cholinergic neuron-like cell shape. Further, it was confirmed that MSCs treated with the inflammatory conditions of the present disclosure secreted acetylcholine to inhibit activated immune cells through nAChRα7, which is an increased nicotinic cholinergic receptor on the surface of immune cells. These properties can be used for the prevention or treatment of immune diseases or inflammatory diseases.

From the foregoing, it will be appreciated that various embodiments of the present disclosure have been described herein for purposes of illustration and that various modifications may be made without departing from the scope and spirit of the present disclosure. Accordingly, the various embodiments disclosed herein are not intended to be limiting, with the true scope and spirit being indicated by the following claims.

## Claims

1. A pharmaceutical composition for preventing or treating an immune disease or an inflammatory disease, the composition comprising an inflammatory stimulated mesenchymal stem cell.

2. The composition according to claim 1, wherein the mesenchymal stem cell is isolated by a subfractionation culturing method.

3. The composition according to claim 1, wherein the mesenchymal stem cell expresses CD29, CD44, CD49f, CD73, CD90, CD105, CD146, HLA-class I (HLA-I) and Oct4.

4. The composition according to claim 1, wherein the inflammatory stimulated mesenchymal stem cell is cultured under at least one culture condition selected from the group consisting of mixed lymphocyte reaction (MLR), mitosis-promoting substance (mitogen) treatment and cytokine treatment conditions.

5. The composition according to claim 4, wherein the mitosis-promoting substance includes at least one selected from the group consisting of phytohemagglutinin (PHA), concanavalin A (Con A), pokeweed mitogen (PWM), lipopolysaccharide, streptolysin S, a mercury compound and an anti-lymphocyte antibody.

6. The composition according to claim 1, wherein the inflammatory stimulated mesenchymal stem cell is transformed to have a cholinergic neuron-like phenotype.

7. The composition according to claim 1, wherein the inflammatory stimulated mesenchymal stem cell secretes acetylcholine.

8. The composition according to claim 1, wherein the immune disease or inflammatory disease includes at least one selected from the group consisting of autoimmune diseases, transplant rejection, arthritis, graft-versus-host-disease, bacterial infection, sepsis and inflammation.

9. The composition according to claim 8, wherein the autoimmune diseases includes at least one selected from the group consisting of Crohn's disease, erythema, atopic dermatitis, rheumatoid arthritis, Hashimoto's thyroiditis, pernicious anemia, Addison's disease, type 1 diabetes, lupus, chronic fatigue syndrome, fibromyalgia, hypothyroidism, hyperthyroidism, scleroderma, Behcet's disease, inflammatory bowel disease, multiple sclerosis, myasthenia gravis, Meniere's syndrome, Guillain-Barre syndrome, Sjogren's syndrome, vitiligo, endometriosis, psoriasis, vitiligo, systemic scleroderma, asthma and ulcerative colitis.

10. A method of preparing a mesenchymal stem cell for preventing or treating an immune disease or an inflammatory disease, the method comprising culturing the mesenchymal stem cell with inflammatory stimulation.

11. The method according to claim 10, wherein the inflammatory stimulation includes at least one selected from the group consisting of mixed lymphocyte reaction (MLR) condition, mitosis-promoting substance (mitogen) treatment and cytokine treatment.

12. The method according to claim 11, wherein the mitosis-promoting substance includes at least one selected from the group consisting of phytohemagglutinin (PHA), concanavalin A (Con A), pokeweed mitogen (PWM), lipopolysaccharide, streptolysin S, a mercury compound and an anti-lymphocyte antibody.

13. The method according to claim 10, wherein the mesenchymal stem cell secretes acetylcholine.


<110>     SCM Lifescience Co., Ltd

<120>     Pharmaceutical composition for prevention or treatment of immune
          diseases and inflammatory diseases comprising immunogenic
          stimulated mesenchymal stem cells

<130>     1-4

<160>     34

<170>     KoPatentIn 3.0

<210>     1
<211>     24
<212>     DNA
<213>     Artificial Sequence

<220>
<223>     Forward Primer of MAP2


<400>     1
taagtttgga gcaagcagtt acag                                              24


<210>     2
<211>     24
<212>     DNA
<213>     Artificial Sequence

<220>
<223>     Reverse Primer of MAP2


<400>     2
ttctctccat acacttttgg atca                                              24


<210>     3
<211>     20
<212>     DNA
<213>     Artificial Sequence

<220>
<223>     Forward Primer of Tuj1


<400>     3
aacgaggcct cttctcacaa                                                   20


<210>     4
<211>     20
<212>     DNA
<213>     Artificial Sequence

<220>
<223>     Reverse Primer of Tuj1


<400>     4
cgataccagg tggttgaggt                                                   20

<210> 5
<211> 19
<212> DNA
<213> Artificial Sequence

<220>
<223> Forward Primer of GFAP


<400> 5
gagtaccagt acctgaaga 19


<210> 6
<211> 20
<212> DNA
<213> Artificial Sequence

<220>
<223> Reverse Primer of GFAP


<400> 6
ttcaccacga tgttcctctt 20


<210> 7
<211> 24
<212> DNA
<213> Artificial Sequence

<220>
<223> Forward Primer of NF-M


<400> 7
tttggtttcc tctatgatct cctc 24


<210> 8
<211> 24
<212> DNA
<213> Artificial Sequence

<220>
<223> Reverse Primer of NF-M


<400> 8
agatggctct ggatatagaa atcg 24


<210> 9
<211> 24
<212> DNA
<213> Artificial Sequence

<220>
<223> Forward Primer of nestin


<400> 9

tccagagctg tcaatgactc taag                                                24

<210>    10
<211>    24
<212>    DNA
<213>    Artificial Sequence

<220>
<223>    Reverse Primer of nestin

<400>    10
gaccactcca gtttagaggc taag                                                24

<210>    11
<211>    20
<212>    DNA
<213>    Artificial Sequence

<220>
<223>    Forward Primer of TrkA

<400>    11
gaagagtggt ctccgtttcg                                                     20

<210>    12
<211>    20
<212>    DNA
<213>    Artificial Sequence

<220>
<223>    Reverse Primer of TrkA

<400>    12
ctgactgctc cagctctgtg                                                     20

<210>    13
<211>    20
<212>    DNA
<213>    Artificial Sequence

<220>
<223>    Forward Primer of TrkB

<400>    13
atcccttcca cagacgtcac                                                     20

<210>    14
<211>    20
<212>    DNA
<213>    Artificial Sequence

<220>
<223>    Reverse Primer of TrkB

<400>    14
tcctgctcag gacagaggtt                                                    20


<210>    15
<211>    20
<212>    DNA
<213>    Artificial Sequence

<220>
<223>    Forward Primer of TrkC


<400>    15
acaagatgct tgtggctgtg                                                    20


<210>    16
<211>    20
<212>    DNA
<213>    Artificial Sequence

<220>
<223>    Reverse Primer of TrkC


<400>    16
gggccctgag gaacttattc                                                    20


<210>    17
<211>    20
<212>    DNA
<213>    Artificial Sequence

<220>
<223>    Forward Primer of p75NTR


<400>    17
agccttcaag aggtggaaca                                                    20


<210>    18
<211>    20
<212>    DNA
<213>    Artificial Sequence

<220>
<223>    Reverse Primer of p75NTR


<400>    18
ctgcacagac tctccacgag                                                    20


<210>    19
<211>    20
<212>    DNA
<213>    Artificial Sequence

<220>

<223>    Forward Primer of NGF

<400>    19
atacaggcgg aaccacactc                                                    20

<210>    20
<211>    20
<212>    DNA
<213>    Artificial Sequence

<220>
<223>    Reverse Primer of NGF

<400>    20
gtctgtggcg gtggtcttat                                                    20

<210>    21
<211>    20
<212>    DNA
<213>    Artificial Sequence

<220>
<223>    Forward Primer of BDNF

<400>    21
tggctgacac tttcgaacac                                                    20

<210>    22
<211>    20
<212>    DNA
<213>    Artificial Sequence

<220>
<223>    Reverse Primer of BDNF

<400>    22
cttatgaatc gccagccaat                                                    20

<210>    23
<211>    17
<212>    DNA
<213>    Artificial Sequence

<220>
<223>    Forward Primer of nAchR-alpha-3

<400>    23
ccatgtctca gctggtg                                                       17

<210>    24
<211>    18
<212>    DNA
<213>    Artificial Sequence

<220>
<223>    Reverse Primer of nAchR-alpha-3

<400>    24
gtccttgagg ttcatgga                                                    18

<210>    25
<211>    18
<212>    DNA
<213>    Artificial Sequence

<220>
<223>    Forward Primer of nAchR-alpha-5

<400>    25
gataatgcag atggacgt                                                    18

<210>    26
<211>    18
<212>    DNA
<213>    Artificial Sequence

<220>
<223>    Reverse Primer of nAchR-alpha-5

<400>    26
tgatggtatg atctcttc                                                    18

<210>    27
<211>    22
<212>    DNA
<213>    Artificial Sequence

<220>
<223>    Forward Primer of nAchR-alpha-7

<400>    27
cccggcaaga ggagtgaaag gt                                               22

<210>    28
<211>    20
<212>    DNA
<213>    Artificial Sequence

<220>
<223>    Reverse Primer of nAchR-alpha-7

<400>    28
ccgggcctct tcattcgcag                                                  20

<210>    29
<211>    20

<212>    DNA
<213>    Artificial Sequence

<220>
<223>    Forward Primer of nAchR-alpha-9


<400>    29
ctacaatggc aatcaggtgg                                                    20


<210>    30
<211>    19
<212>    DNA
<213>    Artificial Sequence

<220>
<223>    Reverse Primer of nAchR-alpha-9


<400>    30
atgatggtca acgcagtgg                                                     19


<210>    31
<211>    17
<212>    DNA
<213>    Artificial Sequence

<220>
<223>    Forward Primer of nAchR-beta-2


<400>    31
cagctcatca gtgtgca                                                       17


<210>    32
<211>    17
<212>    DNA
<213>    Artificial Sequence

<220>
<223>    Reverse Primer of nAchR-beta-2


<400>    32
gtgcggtcgt aggtcca                                                       17


<210>    33
<211>    24
<212>    DNA
<213>    Artificial Sequence

<220>
<223>    Forward Primer of GAPDH


<400>    33
gtcatccatg acaactttgg tatc                                               24

<210>    34
<211>    24
<212>    DNA
<213>    Artificial Sequence

<220>
<223>    Reverse Primer of GAPDH


<400>    34
ctgtagccaa attcgttgtc atac                                              24